# EUROPEAN PATENT APPLICATION

(11) **EP 3 904 877 A1**
(43) Date of publication of application: **03.11.2021**
(21) Application number: 21170874.8
(22) Date of filing: 28.04.2021
(51) Int. Cl.: G01N 33/543

(54) **LATERAL FLOW DETECTION DEVICE FOR DETECTING A CORONOVIRUS BY IMMUNOASSAY**

(30) Priority: 30.04.2020 US 202063018024 P; 18.02.2021 CN 202110063719
(71) Applicant: Hangzhou Biotest Biotech Co., Ltd., Hangzhou, Zhejiang 311100 (CN)
(72) Inventor: Ye, Chunsheng, Hangzhou (CN); Zhang, Zhenxing, Hangzhou (CN); Wang, Zhenyu, Hangzhou (CN); Hou, Luna, Hangzhou (CN)
(74) Representative: Handley, Matthew Edward

(57) **Abstract**

The present invention provides a lateral flow test device for detecting a coronavirus antibody by immunoassay; the test device includes three lateral flow test strips; a first test strip is directed to the antibody detection of a N full-length protein and/or an S full-length protein antigens/antigen; and a second test strip is directed to the antibody detection of an S-RBD-site protein antigen, and both of the first strip and the second strip are combined to detect novel coronavirus IgG and IgM antibodies, which can practically reduce the possibility of missing detection and wrong detection; further, a third test strip is directed to the detection of a neutralizing antibody at an S-RBD site to rapidly detect the neutralizing antibody having protection effect, thus further helping the prevention of missing detection and helping patients to perform self-detection and judge the situations of recovery, or vaccine immunity.

SEQUENCE LISTING

<110>  Hangzhou Biotest Biotech Co., LTD.

<120>  LATERAL FLOW DETECTION DEVICE FOR DETECTING A CORONAVIRUS BY
       IMMUNOASSAY

<130>  MEH/117919EP1

<150>  CN 202110063719.X
<151>  2021-02-18

<150>  US 63/018,024
<151>  2020-04-30

<160>  3

<170>  PatentIn version 3.5

<210>  1
<211>  272
<212>  PRT
<213>  Coronavirus

<400>  1

Met Pro Asn Ile Thr Asn Leu Cys Pro Phe Gly Glu Val Phe Asn Ala
1               5                   10                  15

Thr Arg Phe Ala Ser Val Tyr Ala Trp Asn Arg Lys Arg Ile Ser Asn
            20                  25                  30

Cys Val Ala Asp Tyr Ser Val Leu Tyr Asn Ser Ala Ser Phe Ser Thr
        35                  40                  45

Phe Lys Cys Tyr Gly Val Ser Pro Thr Lys Leu Asn Asp Leu Cys Phe
    50                  55                  60

Thr Asn Val Tyr Ala Asp Ser Phe Val Ile Arg Gly Asp Glu Val Arg
65                  70                  75                  80

Gln Ile Ala Pro Gly Gln Thr Gly Lys Ile Ala Asp Tyr Asn Tyr Lys
                85                  90                  95

Leu Pro Asp Asp Phe Thr Gly Cys Val Ile Ala Trp Asn Ser Asn Asn
            100                 105                 110

Leu Asp Ser Lys Val Gly Gly Asn Tyr Asn Tyr Leu Tyr Arg Leu Phe
        115                 120                 125

Arg Lys Ser Asn Leu Lys Pro Phe Glu Arg Asp Ile Ser Thr Glu Ile
    130                 135                 140

Tyr Gln Ala Gly Ser Thr Pro Cys Asn Gly Val Glu Gly Phe Asn Cys
145                 150                 155                 160

Tyr Phe Pro Leu Gln Ser Tyr Gly Phe Gln Pro Thr Asn Gly Val Gly
                165                 170                 175

Tyr Gln Pro Tyr Arg Val Val Val Leu Ser Phe Glu Leu Leu His Ala
            180                 185                 190

Pro Ala Thr Val Cys Gly Pro Lys Lys Ser Thr Asn Leu Val Lys Asn
        195                 200                 205

Lys Cys Val Asn Phe Asn Phe Asn Gly Leu Thr Gly Thr Gly Val Leu
    210                 215                 220

Thr Glu Ser Asn Lys Lys Phe Leu Pro Phe Gln Gln Phe Gly Arg Asp
225                 230                 235                 240

Ile Ala Asp Thr Thr Asp Ala Val Arg Asp Pro Gln Thr Leu Glu Ile
                245                 250                 255

Leu Asp Ile Thr Pro Cys Ser Phe His His His His His His His His
            260                 265                 270

<210>  2
<211>  425
<212>  PRT
<213>  Coronavirus

<400>  2

Met His His His His His His Ser Asp Asn Gly Pro Gln Asn Gln Arg
1               5                   10                  15

Asn Ala Pro Arg Ile Thr Phe Gly Gly Pro Ser Asp Ser Thr Gly Ser
            20                  25                  30

Asn Gln Asn Gly Glu Arg Ser Gly Ala Arg Ser Lys Gln Arg Arg Pro
        35                  40                  45

Gln Gly Leu Pro Asn Asn Thr Ala Ser Trp Phe Thr Ala Leu Thr Gln
    50                  55                  60

His Gly Lys Glu Asp Leu Lys Phe Pro Arg Gly Gln Gly Val Pro Ile
65                  70                  75                  80

Asn Thr Asn Ser Ser Pro Asp Asp Gln Ile Gly Tyr Tyr Arg Arg Ala
                85                  90                  95

Thr Arg Arg Ile Arg Gly Gly Asp Gly Lys Met Lys Asp Leu Ser Pro
            100                 105                 110

Arg Trp Tyr Phe Tyr Tyr Leu Gly Thr Gly Pro Glu Ala Gly Leu Pro
        115                 120                 125

Tyr Gly Ala Asn Lys Asp Gly Ile Ile Trp Val Ala Thr Glu Gly Ala
    130                 135                 140

Leu Asn Thr Pro Lys Asp His Ile Gly Thr Arg Asn Pro Ala Asn Asn
145                 150                 155                 160

Ala Ala Ile Val Leu Gln Leu Pro Gln Gly Thr Thr Leu Pro Lys Gly
                165                 170                 175

Phe Tyr Ala Glu Gly Ser Arg Gly Gly Ser Gln Ala Ser Ser Arg Ser
            180                 185                 190

Ser Ser Arg Ser Arg Asn Ser Ser Arg Asn Ser Thr Pro Gly Ser Ser
        195                 200                 205

Arg Gly Thr Ser Pro Ala Arg Met Ala Gly Asn Gly Gly Asp Ala Ala
    210                 215                 220

Leu Ala Leu Leu Leu Leu Asp Arg Leu Asn Gln Leu Glu Ser Lys Met
225                 230                 235                 240

Ser Gly Lys Gly Gln Gln Gln Gln Gly Gln Thr Val Thr Lys Lys Ser

245                 250                 255

Ala Ala Glu Ala Ser Lys Lys Pro Arg Gln Lys Arg Thr Ala Thr Lys
        260                 265                 270

Ala Tyr Asn Val Thr Gln Ala Phe Gly Arg Arg Gly Pro Glu Gln Thr
    275                 280                 285

Gln Gly Asn Phe Gly Asp Gln Glu Leu Ile Arg Gln Gly Thr Asp Tyr
    290                 295                 300

Lys His Trp Pro Gln Ile Ala Gln Phe Ala Pro Ser Ala Ser Ala Phe
305                 310                 315                 320

Phe Gly Met Ser Arg Ile Gly Met Glu Val Thr Pro Ser Gly Thr Trp
                325                 330                 335

Leu Thr Tyr Thr Gly Ala Ile Lys Leu Asp Asp Lys Asp Pro Asn Phe
        340                 345                 350

Lys Asp Gln Val Ile Leu Leu Asn Lys His Ile Asp Ala Tyr Lys Thr
    355                 360                 365

Phe Pro Pro Thr Glu Pro Lys Lys Asp Lys Lys Lys Lys Ala Asp Glu
    370                 375                 380

Thr Gln Ala Leu Pro Gln Arg Gln Lys Lys Gln Gln Thr Val Thr Leu
385                 390                 395                 400

Leu Pro Ala Ala Asp Leu Asp Asp Phe Ser Lys Gln Leu Gln Gln Ser
                405                 410                 415

Met Ser Ser Ala Asp Ser Thr Gln Ala
        420                 425

<210>  3
<211>  1282
<212>  PRT
<213>  Coronavirus

<400>  3

Met Phe Leu Leu Thr Thr Lys Arg Thr Met Phe Val Phe Leu Val Leu
1               5                   10                  15

Leu Pro Leu Val Ser Ser Gln Cys Val Asn Leu Thr Thr Arg Thr Gln
            20                  25                  30

Leu Pro Pro Ala Tyr Thr Asn Ser Phe Thr Arg Gly Val Tyr Tyr Pro
        35                  40                  45

Asp Lys Val Phe Arg Ser Ser Val Leu His Ser Thr Gln Asp Leu Phe
    50                  55                  60

Leu Pro Phe Phe Ser Asn Val Thr Trp Phe His Ala Ile His Val Ser
65                  70                  75                  80

Gly Thr Asn Gly Thr Lys Arg Phe Asp Asn Pro Val Leu Pro Phe Asn
                85                  90                  95

Asp Gly Val Tyr Phe Ala Ser Thr Glu Lys Ser Asn Ile Ile Arg Gly
            100                 105                 110

Trp Ile Phe Gly Thr Thr Leu Asp Ser Lys Thr Gln Ser Leu Leu Ile
        115                 120                 125

Val Asn Asn Ala Thr Asn Val Val Ile Lys Val Cys Glu Phe Gln Phe
    130                 135                 140

Cys Asn Asp Pro Phe Leu Gly Val Tyr Tyr His Lys Asn Asn Lys Ser
145                 150                 155                 160

Trp Met Glu Ser Glu Phe Arg Val Tyr Ser Ser Ala Asn Asn Cys Thr
                165                 170                 175

Phe Glu Tyr Val Ser Gln Pro Phe Leu Met Asp Leu Glu Gly Lys Gln
            180                 185                 190

Gly Asn Phe Lys Asn Leu Arg Glu Phe Val Phe Lys Asn Ile Asp Gly
        195                 200                 205

Tyr Phe Lys Ile Tyr Ser Lys His Thr Pro Ile Asn Leu Val Arg Asp
    210                 215                 220

Leu Pro Gln Gly Phe Ser Ala Leu Glu Pro Leu Val Asp Leu Pro Ile
225                 230                 235                 240

Gly Ile Asn Ile Thr Arg Phe Gln Thr Leu Leu Ala Leu His Arg Ser
                245                 250                 255

Tyr Leu Thr Pro Gly Asp Ser Ser Ser Gly Trp Thr Ala Gly Ala Ala
            260                 265                 270

Ala Tyr Tyr Val Gly Tyr Leu Gln Pro Arg Thr Phe Leu Leu Lys Tyr
        275                 280                 285

Asn Glu Asn Gly Thr Ile Thr Asp Ala Val Asp Cys Ala Leu Asp Pro
    290                 295                 300

Leu Ser Glu Thr Lys Cys Thr Leu Lys Ser Phe Thr Val Glu Lys Gly
305                 310                 315                 320

Ile Tyr Gln Thr Ser Asn Phe Arg Val Gln Pro Thr Glu Ser Ile Val
                325                 330                 335

Arg Phe Pro Asn Ile Thr Asn Leu Cys Pro Phe Gly Glu Val Phe Asn
            340                 345                 350

Ala Thr Arg Phe Ala Ser Val Tyr Ala Trp Asn Arg Lys Arg Ile Ser
        355                 360                 365

Asn Cys Val Ala Asp Tyr Ser Val Leu Tyr Asn Ser Ala Ser Phe Ser
    370                 375                 380

Thr Phe Lys Cys Tyr Gly Val Ser Pro Thr Lys Leu Asn Asp Leu Cys
385                 390                 395                 400

Phe Thr Asn Val Tyr Ala Asp Ser Phe Val Ile Arg Gly Asp Glu Val
                405                 410                 415

Arg Gln Ile Ala Pro Gly Gln Thr Gly Lys Ile Ala Asp Tyr Asn Tyr
            420                 425                 430

Lys Leu Pro Asp Asp Phe Thr Gly Cys Val Ile Ala Trp Asn Ser Asn
        435                 440                 445

Asn Leu Asp Ser Lys Val Gly Gly Asn Tyr Asn Tyr Leu Tyr Arg Leu
    450                 455                 460

Phe Arg Lys Ser Asn Leu Lys Pro Phe Glu Arg Asp Ile Ser Thr Glu
465                 470                 475                 480

Ile Tyr Gln Ala Gly Ser Thr Pro Cys Asn Gly Val Glu Gly Phe Asn
                485                 490                 495

Cys Tyr Phe Pro Leu Gln Ser Tyr Gly Phe Gln Pro Thr Asn Gly Val
            500                 505                 510

Gly Tyr Gln Pro Tyr Arg Val Val Val Leu Ser Phe Glu Leu Leu His
        515                 520                 525

Ala Pro Ala Thr Val Cys Gly Pro Lys Lys Ser Thr Asn Leu Val Lys
    530                 535                 540

Asn Lys Cys Val Asn Phe Asn Phe Asn Gly Leu Thr Gly Thr Gly Val
545                 550                 555                 560

Leu Thr Glu Ser Asn Lys Lys Phe Leu Pro Phe Gln Gln Phe Gly Arg
                565                 570                 575

Asp Ile Ala Asp Thr Thr Asp Ala Val Arg Asp Pro Gln Thr Leu Glu
            580                 585                 590

Ile Leu Asp Ile Thr Pro Cys Ser Phe Gly Gly Val Ser Val Ile Thr
        595                 600                 605

Pro Gly Thr Asn Thr Ser Asn Gln Val Ala Val Leu Tyr Gln Asp Val
    610                 615                 620

Asn Cys Thr Glu Val Pro Val Ala Ile His Ala Asp Gln Leu Thr Pro
625                 630                 635                 640

Thr Trp Arg Val Tyr Ser Thr Gly Ser Asn Val Phe Gln Thr Arg Ala
                645                 650                 655

Gly Cys Leu Ile Gly Ala Glu His Val Asn Asn Ser Tyr Glu Cys Asp
            660                 665                 670

Ile Pro Ile Gly Ala Gly Ile Cys Ala Ser Tyr Gln Thr Gln Thr Asn
        675                 680                 685

Ser Pro Arg Arg Ala Arg Ser Val Ala Ser Gln Ser Ile Ile Ala Tyr
    690                 695                 700

Thr Met Ser Leu Gly Ala Glu Asn Ser Val Ala Tyr Ser Asn Asn Ser
705                 710                 715                 720

Ile Ala Ile Pro Thr Asn Phe Thr Ile Ser Val Thr Thr Glu Ile Leu
                725                 730                 735

Pro Val Ser Met Thr Lys Thr Ser Val Asp Cys Thr Met Tyr Ile Cys
            740                 745                 750

Gly Asp Ser Thr Glu Cys Ser Asn Leu Leu Leu Gln Tyr Gly Ser Phe
        755                 760                 765

Cys Thr Gln Leu Asn Arg Ala Leu Thr Gly Ile Ala Val Glu Gln Asp
    770                 775                 780

Lys Asn Thr Gln Glu Val Phe Ala Gln Val Lys Gln Ile Tyr Lys Thr
785                 790                 795                 800

Pro Pro Ile Lys Asp Phe Gly Gly Phe Asn Phe Ser Gln Ile Leu Pro
                805                 810                 815

Asp Pro Ser Lys Pro Ser Lys Arg Ser Phe Ile Glu Asp Leu Leu Phe
            820                 825                 830

Asn Lys Val Thr Leu Ala Asp Ala Gly Phe Ile Lys Gln Tyr Gly Asp
        835                 840                 845

Cys Leu Gly Asp Ile Ala Ala Arg Asp Leu Ile Cys Ala Gln Lys Phe
    850                 855                 860

Asn Gly Leu Thr Val Leu Pro Pro Leu Leu Thr Asp Glu Met Ile Ala
865                 870                 875                 880

Gln Tyr Thr Ser Ala Leu Leu Ala Gly Thr Ile Thr Ser Gly Trp Thr
                885                 890                 895

Phe Gly Ala Gly Ala Ala Leu Gln Ile Pro Phe Ala Met Gln Met Ala
            900                 905                 910

Tyr Arg Phe Asn Gly Ile Gly Val Thr Gln Asn Val Leu Tyr Glu Asn
        915                 920                 925

Gln Lys Leu Ile Ala Asn Gln Phe Asn Ser Ala Ile Gly Lys Ile Gln
    930                 935                 940

Asp Ser Leu Ser Ser Thr Ala Ser Ala Leu Gly Lys Leu Gln Asp Val
945                 950                 955                 960

Val Asn Gln Asn Ala Gln Ala Leu Asn Thr Leu Val Lys Gln Leu Ser
                965                 970                 975

Ser Asn Phe Gly Ala Ile Ser Ser Val Leu Asn Asp Ile Leu Ser Arg
            980                 985                 990

Leu Asp Lys Val Glu Ala Glu Val  Gln Ile Asp Arg Leu  Ile Thr Gly
        995                 1000                1005

Arg Leu  Gln Ser Leu Gln Thr  Tyr Val Thr Gln Gln  Leu Ile Arg
    1010                1015                1020

Ala Ala  Glu Ile Arg Ala Ser  Ala Asn Leu Ala Ala  Thr Lys Met
    1025                1030                1035

Ser Glu  Cys Val Leu Gly Gln  Ser Lys Arg Val Asp  Phe Cys Gly
    1040                1045                1050

Lys Gly  Tyr His Leu Met Ser  Phe Pro Gln Ser Ala  Pro His Gly
    1055                1060                1065

Val Val  Phe Leu His Val Thr  Tyr Val Pro Ala Gln  Glu Lys Asn
    1070                1075                1080

Phe Thr Thr Ala Pro Ala Ile Cys His Asp Gly Lys Ala His Phe
    1085                1090                1095

Pro Arg Glu Gly Val Phe Val Ser Asn Gly Thr His Trp Phe Val
    1100                1105                1110

Thr Gln Arg Asn Phe Tyr Glu Pro Gln Ile Ile Thr Thr Asp Asn
    1115                1120                1125

Thr Phe Val Ser Gly Asn Cys Asp Val Val Ile Gly Ile Val Asn
    1130                1135                1140

Asn Thr Val Tyr Asp Pro Leu Gln Pro Glu Leu Asp Ser Phe Lys
    1145                1150                1155

Glu Glu Leu Asp Lys Tyr Phe Lys Asn His Thr Ser Pro Asp Val
    1160                1165                1170

Asp Leu Gly Asp Ile Ser Gly Ile Asn Ala Ser Val Val Asn Ile
    1175                1180                1185

Gln Lys Glu Ile Asp Arg Leu Asn Glu Val Ala Lys Asn Leu Asn
    1190                1195                1200

Glu Ser Leu Ile Asp Leu Gln Glu Leu Gly Lys Tyr Glu Gln Tyr
    1205                1210                1215

Ile Lys Trp Pro Trp Tyr Ile Trp Leu Gly Phe Ile Ala Gly Leu
    1220                1225                1230

Ile Ala Ile Val Met Val Thr Ile Met Leu Cys Cys Met Thr Ser
    1235                1240                1245

Cys Cys Ser Cys Leu Lys Gly Cys Cys Ser Cys Gly Ser Cys Cys
    1250                1255                1260

Lys Phe Asp Glu Asp Asp Ser Glu Pro Val Leu Lys Gly Val Lys
    1265                1270                1275

Leu His  Tyr Thr
    1280

## Description

### Cross-Reference

The present invention, as a continuation-in-part of the US Application No. 17/230,302, filed on April 14, 2021, which claims the priority of a US provisional application, Application No.: 63/018,024 filed on April 30, 2020; and claims the priority of a Chinese prior application, Application No.: 202110063719X filed on February 18, 2021. All aforementioned patent applications are hereby incorporated by reference.

### Technical Field

The present invention belongs to the field of measurement for biotechnique, and relates to a lateral flow test device for detecting a coronavirus antibody by immunoassay.

### Background

A terrible cold (COVID-19) caused by a novel coronavirus broke out in Wuhan 2019; and the pneumonia caused by virus infection is named coronavirus disease 2019, namely, COVID-19 by National Health Commission.COVID-19 spreads very rapidly. The epidemic spreads to the whole country rapidly and sweeps across the globe in just three months. More than 200 countries have reported 2 million confirmed cases above. Due to high infectivity, high pathogenicity, high rate of severe illness, and high mortality, such kind of coronavirus causes serious damages to the global economy, society, health and other aspects.

With the understanding to biological characteristics of novel coronavirus, certain effect has been achieved in the treatment of COVID-19 currently, and moreover, China has basically controlled the outbreak of COVID-19. More than 90% patients have been discharged from hospital at home; but partial patients in many places, such as Guangdong, Sichuan, Hubei, and Hunan have had a fever, and a positive result of nucleic acid testing after discharge once again. It has been not clear whether the discharge standard is not strict or the virus relapses really on earth. Partial severe/critical COVID-19 patients get an improvement after treatment, but advanced age, immunocompromise, structural lung disease, or change of lung structure caused by pulmonary fibrosis due to a severe condition results in incomplete blood circulation and hemoperfusion, so that partial concealed virus are not completely cleaned, or cells still carry virus; and the virus expelling capacity of the body has been not up to the virus amount required by a positive result of nucleic acid testing; and if immunity of the organism is low, the virus not killed completely may relapse and reappear to cause the disease relapse. At this stage, the asymptomatic brings a great challenge to the epidemic prevention and control, whether COVID-19 asymptomatic carriers cause the rebound of the epidemic in China has become a hot topic widely concerned by the society.

Currently, the method for detecting COVID-19 mainly includes nucleic acid assay and immunological detection; the nucleic acid testing is featured by early diagnosis, high sensitivity and specificity and thus is a "golden standard" to confirm COVID-19. Due to a relatively high requirement of sample collection, RNA extraction and test equipment conditions, a false-negative result easily appears, it is in urgent need of a rapid immunological detection method to make up the shortcoming of missed diagnosis caused by the false negative of nucleic acid detection.

2019-nCoV includes coronavirus spike proteins (Spike, S), envelope proteins (Envelope, E), membrane proteins (Membrane, M), Nucleoprotein (N) and other structures. S protein can bind an ACE2 receptor on the surface of a host cell,and is an important structural protein to mediate virus entry into a cell, and is also a major antigen to induce a neutralizing antibody. Usually, S protein can be cut into two parts, namely, S1 and S2; S1 mediates virus's adhering, S2 mediates membrane fusion, and RBD is a receptor binding domain to an S1 structure.

CN111505277A discloses a detection kit of novel coronavirus; and the kit is coated with a RBD and N antigen combination for the detection of an IgG antibody. RBD, as an accurate-site protein, in the kit is combined with nucleocapsid N protein to easily cause a cross impact, moreover, the only detection of the IgG antibody further expands the possibility of missing detection and wrong detection.

Neutralizing antibody is a kind of soluble protein secreted by adaptive immune response cells, and has the functions of identifying the virus surface protein and blocking out its binding to a specific receptor of cell surface, thus exerting the antiviral effect. The amount of the neutralizing antibody is an important index to determine the immune protection effect of a vaccine, and is an important basis to evaluate the vaccine and control the quality. Human body produces protective antibodies, namely, neutralizing antibodies through immune response after vaccination; and the neutralizing antibody is subjected to titre determination to judge the clinical efficacy of the vaccine. Therefore, the detection for a neutralizing antibody can be applied in research, development and evaluation of a vaccine, as well as for the evaluation of autoimmune effect of an individual after the vaccine is successfully developed and formally applied in the public. Furthermore, the detection may be used in cured COVID-19 patients to judge whether there is a risk of reinfection.

### Summary of the Invention

To make up the shortcomings of the prior art, the present invention provides a rapid, simple and reliable lateral flow test device for detecting a novel coronavirus antibody by immunoassay. The test device includes three lateral flow test strips; a first test strip is directed to the antibody detection of a N protein (full-length) and/or an S protein (full-length) antigens/antigen; and a second test strip is directed to the antibody detection of an S-RBD-site protein, which covers each site of the novel coronavirus which may render a human body to produce an antibody more thoroughly, thus further promoting the detection ratio the antibody by serology to avoid missing detection; further, a third test strip is used to rapidly detect a neutralizing antibody having protection effect. On the one hand, such kind of test combination and method can further help avoiding missing detection based on the detection of the first and the second test strips; on the other hand, can help patients self-detecting and judging the recovery and vaccine immunity condition, which provides more visual and reliable self-checking means for the patients to perform self-screening, control the progression and recovery of disease, or control the effect of the vaccine, and judge whether of requiring supplementary vaccination. Moreover, the test device has the following advantages of rapid detection speed and simple operation, is free from professional instrument and personnel, and easy to clinical popularization and application.

For the antibody detection of a novel coronavirus, on the one hand, it needs to further reduce the possibility of leak and wrong detection to improve the detection sensitivity; on the other hand, it needs to detect multiple kinds of antibodies respectively to analyze the content of neutralizing antibodies and other antibodies, thus judging the progression of disease, judging whether of getting recovery, and helping judging the vaccine immunity condition after vaccination, and helping whether of a risk of reinfection. After being infected with novel coronavirus, patients can judge whether of being in the stage of recovery or in the stage of aggravation by self-detecting whether the ratio of neutralizing antibodies is in upward or down trend at fixed period per day at home, thus providing referential experiences for the subsequent therapeutic measures and helping preventing the spread of the epidemic.

The novel coronavirus binds to an angiotensin converting enzyme 2 (ACE2) receptor of human cells mainly by a spike (S) protein on the virus when begins to invade our body, while in practical binding, the S protein is lysed into an S1 subunit and an S2 subunit by a host protease (cysteine protease, trypsin, and the like) rather than a simple S protein is inserted onto ACE2; S1 and S2 will fuse with a receptor binding membrane; of which S1 contains a receptor binding domain RBD; and RBD is just the key to binding ACE2. Therefore, the antibody of S-RBD-site protein and the antibody of the S full-length protein are not always present at the same time; and there is a possibility of missing detection during the process of separately detecting the antibody produced by the S-RBD-site protein antigen or detecting the antibody produced by the S full-length protein antigen separately. Thereby, it is very important to respectively detect the antibodies produced by the S full-length protein and the S-RBD-site protein at the same time. The S1 subunit may be further divided into two relatively independent domains, a N-terminal domain (NTD) and C-terminal domain (CTD) respectively. S1 contains a receptor binding domain (RBD); RBD of the majority of the coronavirus S proteins is located at CTD, for example, SARS-CoV and MERS-CoV. RBD of only a small amount of β coronavirus is located at NTD (usually, NTD binds to saccharide receptors, CTD binds to protein receptors). Based on the mentioned above, it seems that if an antibody of a RBD antigen is detected, it may cause missing detection; and if the detection is combined with monitoring the antibody of the S full-length protein, all antibodies produced by the virus may be detected more thoroughly.

N protein is rich in novel coronavirus, and has high conservative property, and is associated with the duplication of viral genome and the regulation of cell signaling pathway. Therefore, N protein can serve as a detection tool for the diagnosis of novel coronavirus, and is a core material of a rapid diagnostic reagent in immunology. However, N protein comes into play after novel coronavirus invades the body, there is still a risk of missing detection during the process of separately detecting the antibody of the N full-length protein. Therefore, it is very necessary to cooperate with the S full-length protein which comes into play at the beginning of the invasion for combined detection, thus reducing the possibility of missing detection.

Meanwhile, S-RBD site is a receptor binding domain of novel coronavirus, and is the core to binding an ACE2 receptor of human cells, and is an important fragment to determine the infection ability of novel coronavirus. The antibody to the S-RBD-site protein is the most efficient in numerous effective antibodies; the single detection of the antibody of the S-RBD-site protein can improve the detection accuracy and efficiency, and also can be used to identify the production of an effective antibody produced by congenital immunity and/or immune after recovery; and the neutralizing antibody contained therein can directly act on a RBD fragment on the S protein, thus directly preventing the virus from infecting cells. But S or N full-length protein has no such function). As an accurate site protein, the S-RBD-site protein is placed on a test strip with a full-length antigen (for example, S or other N antigens) for detection, and both are treated in a test reaction zone, easily resulting in cross reaction but no complementary effect, which not only reduces the effect of detecting the effective antibody, but also causes missing detection easily. Moreover, the way cannot identify the production of an effective antibody produced by congenital immunity and/or immune after recovery. Congenital immunity refers that after being infected with the virus, a body produces antibodies itself, but shows no symptom, actually, the virus is present in the body, and thus the body may be infectious; while the antibody produced after recovery refers that a body has been infected with the virus and has antibodies, that is, the patient is in the stage of recovery.

Even though the antibody produced by the action of RBD, such kind of antibody includes the antibody produced by the virus infection into our body and also neutralizing antibodies; general detection refers to the detection of total antibodies; therefore, the detection cannot differ neutralizing antibodies from other antibodies.

The neutralizing antibody is detected by the S-RBD site to further help judging the amount of the neutralizing antibodies capable of neutralizing the virus effectively in the antibodies of a patient's body. The above detection is also a monitoring process to judge whether the body produces neutralizing antibodies itself after virus infection, thus judging whether the body is in a state of recovery. For example, during the analysis of detection results per day, if the total amount of antibodies has no obvious changes or has a down trend, but the content of neutralizing antibodies rises day by day, it proves that the patient gets recovery day by one, and the antiviral ability enhances day by day; if the total amount of antibodies has no obvious changes or has an upward trend, but the content of neutralizing antibodies decreases day by day (or the total amount of antibodies rises, but the content of neutralizing antibodies keeps stable), it proves that the virus is still present in the patient's body, moreover, the reproductive capacity of the virus is greater than the production capacity of the neutralizing antibody, and the virus prevails, thus predicting that the disease has been not controlled effectively and is infectious; if the total amount of antibodies rises day by day, and the content of neutralizing antibodies rises day by day as well, it may be the production of effective antibodies after vaccination to gradually enhance the immunity to the virus; and if the total amount of antibodies has a down trend day by day, and the content of neutralizing antibodies decreases day by day as well, it indicates that the immunity reduces, and it is necessary to supplement vaccination.

Moreover, total antibodies are combined with neutralizing antibodies for detection to further prevent the possibility of missing detection. For example, when the RBD antibody is detected negative, but the neutralizing antibody is positive, it indicates that the human body may be vaccinated to have a defense function, may not be considered to be infected with the coronavirus.

The present invention finds that where the first test strip is used to detect the antibody of the N protein (full-length) and/or S protein (full-length) antigen or the full-length antigen, a second test strip is combined to detect the most effective antibody or RBD antigen produced specific to the S-RBD site to bring a complementary effect, thus thoroughly avoiding missing detection; meanwhile, the third test strip is combined to detect the neutralizing antibody produced specific to the S-RBD site, which further helps identifying the asymptomatic, judging the recovery of the patient, and the immunization of the vaccine, thus providing a more reliable on-site test means for the checking of the patient and screening of the asymptomatic.

Antibodies of the first test strip and the second test strip may include IgM and/or IgG antibodies corresponding to these antigens. These antibodies may exist in blood, saliva, lung, throat and other secretions; and the blood includes serum, plasma or a sample of the whole blood. Researches show that after novel coronavirus invades a human body, IgM antibodies appear within 5-7 days firstly, and then IgG antibodies appear within 10-15 days. Therefore, the increase of IgM antibodies hints acute infection recently, and the increase of IgG antibodies hints the previous infection. Two groups of antigen proteins in two test strips provided in the present invention are combined to practically decrease the possibility of missing detection and wrong detection via the further joint detection of the novel coronavirus IgG and IgM antibodies, thus avoiding missing detection thoroughly.

Certainly, the test device provided by the present invention also can be used for detecting the novel coronavirus antigen by adjusting the antibodies in a label area and detection area. For example, the RBD antigen can be detected on a first test strip, and the full-length N or/and S antigens can be detected on a second test strip. The antigen can be detected by an antibody for detecting the antigen or other receptors capable of binding to the antigen.

The antibody detection for the first, second and third test strips is combined to help judging the anti-virus capability more accurately, which provides more professional advices and guidance for the high-risk population to return to work or take part in more social activities.

The three sets of antigen combination in the present invention may be natural antigens, or recombinant antigens expressed by a conventional genetic engineering technology. Moreover, these antigens need to be detected separately instead of being mixed, and the specific meaning of the separate detection will be explained in the present application specifically below.

Therefore, the prevent invention provides the following technical solution:
On the one hand, the present invention provides a lateral flow test device for detecting a coronavirus antibody by immunoassay, where the device includes a first test strip, a second test strip and a third lateral flow test strip; the first lateral flow test strip contains an antibody for detecting an S and/or a N full-length protein antigen of the novel coronavirus; the second lateral flow test strip contains an antibody for detecting an S-RBD-site protein antigen of the novel coronavirus; and the third test strip contains an S-RBD-site protein antigen; and an angiotensin converting enzyme 2.

In some embodiments, the antibody includes IgM or/and IgG antibodies.

In some embodiments, the second lateral flow test strip contains IgM or/and IgG antibodies for detecting an S-RBD-site protein of coronavirus.

In some embodiments, the first lateral flow test strip contains IgM or/and IgG antibodies for detecting an S full-length protein or/and N full-length protein of novel coronavirus. In some embodiments, antibodies are IgM or/and IgG antibodies of the S or N protein. In some embodiments, S and N proteins are mixed to form a detection area. In some embodiments, IgG for detecting N and S proteins is a detection area; IgM for detecting N and S proteins is another detection area; both or either of the detection areas is located at the same test strip. A reagent for detecting the antibody of a RBD antigen is located at another different test strip, and the test strip includes an IgG or IgM antibody.

The S full-length protein has an amino acid sequence as shown in SEQ ID NO.3; the N full-length protein has an amino acid sequence as shown in SEQ ID NO.2, and the S-RBD-site protein has an amino acid sequence as shown in SEQ ID NO.1.

On the other hand, the present invention provides a lateral flow test device for detecting a coronavirus antibody by immunoassay, where the detection device includes a first test strip, a second test strip and a third test strip; the first test strip contains an S and/or a N full-length protein antigen; the second test strip contains an S-RBD-site protein antigen; and the third test strip contains an S-RBD-site protein antigen; and an angiotensin converting enzyme 2.
1. QHR63250-nCov-S RBD-263aa: 
MPNITNLCPFGEVFNATRFASVYAWNRKRISNCVADYSVLYNSASFSTFKCYGVSPTK
LNDLCFTNVYADSFVIRGDEVRQIAPGQTGKIADYNYKLPDDFTGCVIAWNSNNLDSKVG
GNYNYLYRLFRKSNLKPFERDISTEIYQAGSTPCNGVEGFNCYFPLQSYGFQPTNGVGYQP
YRVVVLSFELLHAPATVCGPKKSTNLVKNKCVNFNFNGLTGTGVLTESNKKFLPFQQFGR
 DIADTTDAVRDPQTLEILDITPCSFHHHHHHHH （SEQ ID NO: 1).
2. QHN73817-nCov N-419aa: (N full-length protein) 
MHHHHHHSDNGPQNQRNAPRITFGGPSDSTGSNQNGERSGARSKQRRPQGLPNNTA
SWFTALTQHGKEDLKFPRGQGVPINTNSSPDDQIGYYRRATRRIRGGDGKMKDLSPRWYF
YYLGTGPEAGLPYGANKDGIIWVATEGALNTPKDHIGTRNPANNAAIVLQLPQGTTLPKG
FYAEGSRGGSQASSRSSSRSRNSSRNSTPGSSRGTSPARMAGNGGDAALALLLLDRLNQLE
SKMSGKGQQQQGQTVTKKSAAEASKKPRQKRTATKAYNVTQAFGRRGPEQTQGNFGDQ
ELIRQGTDYKHWPQIAQFAPSASAFFGMSRIGMEVTPSGTWLTYTGAIKLDDKDPNFKDQ
VILLNKHIDAYKTFPPTEPKKDKKKKADETQALPQRQKKQQTVTLLPAADLDDFSKQLQQ
SMSSADSTQA(SEQ ID NO: 2).
3. Amino acid sequence of S full-length protein

1 mfllttkrtmfvflvllplvssqcvnlttrtqlppaytnsftrgvyypdkvfrssvlhst
61 qdlflpffsnvtwfhaihvsgtngtkrfdnpvlpfndgvyfasteksniirgwifgttld
121sktqsllivnnatnvvikvcefqfcndpflgvyyhknnkswmesefrvyssannctfeyv
181sqpflmdlegkqgnfknlrefvfknidgyfkiyskhtpinlvrdlpqgfsaleplvdlpi
241ginitrfqtllalhrsyltpgdsssgwtagaaayyvgylqprtfllkynengtitdavdc
301aldplsetkctlksftvekgiyqtsnfrvqptesivrfpnitnlcpfgevfnatrfasvy
361awnrkrisncvadysvlynsasfstfkcygvsptklndlcftnvyadsfvirgdevrqia
421pgqtgkiadynyklpddftgcviawnsnnldskvggnynylyrlfrksnlkpferdiste
481pgqtgkiadynyklpddftgcviawnsnnldskvggnynylyrlfrksnlkpferdiste
541 nlvknkcvnfnfngltgtgvltesnkkflpfqqfgrdiadttdavrdpqtleilditpcs
601 fggvsvitpgtntsnqvavlyqdvnctevpvaihadqltptwrvystgsnvfqtragcli
661 gaehvnnsyecdipigagicasyqtqtnsprrarsvasqsiiaytmslgaensvaysnns
721 iaiptnftisvtteilpvsmtktsvdctmyicgdstecsnlllqygsfctqlnraltgia
781 veqdkntqevfaqvkqiyktppikdfggfnfsqilpdpskpskrsfiedllfnkvtlada
841 gfikqygdclgdiaardlicaqkfngltvlppllltdemiaqytsallagtitsgwtfgag
901 aalqipfamqmayrfngigvtqnvlyenqklianqfnsaigkiqdslsstasalgklqdv
961 vnqnaqalntlvkqlssnfgaissvlndilsrldkveaevqidrlitgrlqslqtyvtqq
1021 liraaeirasanlaatkmsecvlgqskrvdfcgkgyhlmsfpqsaphgvvflhvtyvpaq
1081 eknfttapaichdgkahfpregvfvsngthwfvtqrnfyepqiittdntfvsgncdvvig
1141 ivnntvydplqpeldsfkeeldkyfknhtspdvdlgdisginasvvniqkeidrlnevak
1201 nlneslidlqelgkyeqyikwpwyiwlgfiagliaivmvtimlccmtsccsclkgccscg
1261 scckfdeddsepvlkgvklhyt (SEQ ID NO: 3)

Further, the first, or the second test strip, and the third test strip respectively include a sample area, a label area and a detection area which are arranged successively according to a direction of liquid flow; a substance coated in the label area may flow with liquid and the substance needs to be coupled to a marker; the detection area is provided with a detection line; and a substance coated in the detection area needs to be immobilized on the detection line; and the antigen may be coated in the label area.

A substance capable of flowing with liquid on the label area is coupled to a marker, and captured by an antibody or antigen on the detection line after flowing to the detection line, thus forming a colored line. The substance coated by the detection line is fixed. The mark may be fluorescence, or a colored particle, such as, latex, gold particle, or a colored water-soluble substance.

Further, the first test strip or second test strip further contains an anti-human IgG antibody and/or an anti-human IgM antibody; moreover, when the antigen is coated in the label area, the anti-human IgG antibody and/or the anti-human IgM antibody is coated in the detection area. Alternately, when the antigen is coated in the detection area, the anti-human IgG antibody and/or the anti-human IgM antibody is coated in the label area, capable of flowing with the sample.

Further, an anti-human IgG antibody and/or anti-human IgM antibody are immobilized on a detection area of the first and the second test strips; an angiotensin converting enzyme 2 is immobilized on the detection area of the third test strip; and when the antigen is coupled to a marker with colored particles and treated on the detection area; or when the antigen is immobilized on the detection area, the anti-human IgG antibody and/or anti-human IgM antibody and/or the angiotensin converting enzyme 2 are coupled to the marker with colored particles and coated on the label area.Alternately, the neutralizing antibody is detected by a competition law; and an antibody of the S or N full-length protein is detected by a sandwich method or a similar sandwich method.

The anti-human IgG antibody and/or anti-IgM antibody, namely, the IgM or IgG antibody to be detected may be a murine anti-human IgM antibody, a murine anti-human IgG antibody, a rabbit anti-human IgM antibody, a rabbit anti-hum.an IgG antibody, and the like as long as the antibody can specifically capture the antibody IgM or IgG to be detected.

The S full-length protein and/or N full-length protein of the first test strip may be coated in the label area and coupled to a marker, and bonded to IgM or IgG in a sample, and then flows to the detection area with liquid, and is captured and color developed by the anti-human IgG antibody and/or anti-human IgM antibody coated in the detection line, thus obtaining a detection result; on the contrary, when the anti-human IgG antibody and/or anti-human IgM antibody is coated in the label area and coupled to a marker, and the antibody may be bonded to IgM or IgG in a sample, and then flows with liquid to the detection area, and afterwards, is captured and color developed by the S full-length protein antigen and/or N full-length protein antigen coated in the detection line, thus obtaining a detection result.

Similarly, the S-RBD-site protein antigen of the second test strip may be coated in the label area and coupled to a marker, and bonded to IgM or IgG in a sample, and then flows to the detection area with liquid, and is captured and color developed by the anti-human IgG antibody and/or anti-human IgM antibody coated in the detection line, thus obtaining a detection result; on the contrary, when the anti-human IgG antibody and/or anti-human IgM antibody is coated in the label area and coupled to a marker, and the antibody may be bonded to IgM or IgG in a sample, and then flows with liquid to the detection area, and afterwards, is captured and color developed by the S-RBD-site protein antigen coated in the detection line, thus obtaining a detection result.

The S-RBD-site protein antigen of the third test strip may be coated in the label area and coupled to a marker, and bonded to IgM or IgG in a sample, and then flows to the detection area with liquid; while the unbonded S-RBD antigen is captured and color developed by an angiotensin converting enzyme 2 coated in the detection line, thus obtaining a detection result.

Further, the detection areas of the first and second test strips may be provided with one or more detection lines; and the detection line is respectively used for detecting any one or two of IgG and IgM antibodies.

When a detection line is configured, the detection line is used to detect the total amount of IgG and gM, the result is positive as long as one of IgG or IgM is positive; when two detection lines are configured, the detection lines are respectively used to detect IgG and IgM, the result is positive as long as one of IgG or IgM is positive; moreover, the increase of IgM antibodies hints acute infection recently; and the increase of IgG antibodies hints previous infection; when two detection lines are configured, there is another situation in the same test strip, namely, one detection line is used to detect the N full-length protein antibody (including the total amount of IgG and IgM), another one is used to detect the S full-length protein antibody (including the total amount of IgG and IgM).

Certainly, the test device provided by the present invention also can be used for detecting the novel coronavirus antigen by adjusting the type of antibodies in a label area and detection area.At this time, the one or more detection lines can be respectively used to detect different antigens.

Further, the S-RBD-site protein antigen on the second test strip is coated in the label area; the anti-human IgG and anti-human IgM antibodies are respectively coated on the detection lines of the detection area.

Further, the S-RBD-site protein antigen on the second test strip is coated in the label area; the anti-human IgG and anti-human IgM antibodies are respectively coated on the different detection lines of the detection area for respectively detecting IgG and IgM; and for efficiently judging whether there is an effective antibody produced by congenital immunity and/or immunization after recovery, thus further exerting the effect of the second test strip.

Further, when a first antibody of the S full-length protein and/or N full-length protein antibody is coated by the label area on the first test strip, the detection area may immobilize a second antibody against the S full-length protein and/or N full-length protein antibody; or the detection area may also immobilize a second antibody against the first antibody of the S full-length protein and/or N full-length protein antibody.

The N or S full-length protein antibody may be detected via a direct method of double antibodies sandiwich, or an indirect method; and the N or S full-length protein antibody (in a sample, for example, blood) herein may be regarded as an antigen in practice. When the N or S full-length protein antibody is detected, a mark may be coupled to the N or S full-length protein antibody, and a second antibody against the N full-length protein antibody is immobilized in the detection area, which is called the double antibodies sandiwich method. Certainly, a marker may be coupled to a first antibody against the N full-length protein antibody, and a second antibody against the first antibody is immobilized in the detection area, which is called the indirect method, optionally.

Further, in some embodiments, if the test strip 2 is only used to prevent missing detection, the above antibody configuration way may be also taken; when a first antibody of the S-RBD-site protein antibody is coated by the label area, the detection area may immobilize a second antibody against the S full-length protein and/or N full-length protein antibody; or the detection area may also immobilize a second antibody against the first antibody of the S full-length protein and/or N full-length protein antibody.

Further, the detection area is further provided with a quality control line. The label area may be provided with a substance capable of reacting with the substance in the label area to form a labeled complex, and a goat anti-chicken IgY antibody, a goat anti-mouse IgG antibody, and the like are taken.

Further, the first test strip, the second test strip, or the third test strip further comprises a water absorbing area, used for adding a buffer solution.

The water absorbing area may be also called a buffer solution adding area, and the buffer solution may be a buffer solution PBS, and the like.

Further, the coronavirus is novel coronavirus.

In a further aspect, the prevent invention provides use of three groups of antigens in preparing a lateral flow test device for detecting a coronavirus antibody by immunoassay; the first group includes an S full-length protein antigen and/or a N full-length protein antigen of novel coronavirus; the second group includes an S-RBD-site protein antigen of novel coronavirus; the third group includes an S-RBD-site protein antigen and an angiotensin converting enzyme 2 of novel coronavirus; the S full-length protein has an amino acid sequence as shown in SEQ ID NO.3; the N full-length protein has an amino acid sequence as shown in SEQ ID NO.2, and the S-RBD-site protein has an amino acid sequence as shown in SEQ ID NO.1.

The three groups of antigens provided by the present invention can help recognizing the content of total antibodies, and the content of neutralizing antibodies in a sample rapidly, thus helping identifying the recovery and vaccine immunity conditions of the asymptomatic and patients.

The prevent invention provides a method for preparing a lateral flow test device for detecting a novel coronavirus antibody by immunoassay below:
(1) detection area: a nitrocellulose membrane is used, and a buffer solution PBS is used to dissolve antibodies on a detection line, then a membrane-dotting device is used to line out on the nitrocellulose membrane to keep the distance among different antibodies within 3-8 mm, then the nitrocellulose membrane is placed in an oven for drying for further use.
(2) sample area: a sample applying pad is used, and the sample applying pad is made of glass fibre.
(3) label area: an antigen or antibody labeled by a gold particle is prepared, and a labeled mixture is sprayed on a polyester film to be prepared into a label pad.
(4) water absorbing area: a common water-absorbing filter paper is used as a water absorbing pad.
(5) Assembly: one end of the sample applying pad is overlapped on the label pad, and the label pad is overlapped on the nitrocellulose membrane; the nitrocellulose membrane on one end of the quality control line is overlapped by the water-absorbing filter paper to form an entire test strip, and then, the test strip is assembled in a detection card, of which sample loading wells on the detection card are corresponding to the sample applying pad, and the nitrocellulose membrane corresponds to a degree window.

A use method of a lateral flow test device for detecting a novel coronavirus antibody by immunoassay provided by the present invention is as follows:
A proper amount of samples is taken and dropwisely added to a sample area of the test device, after standing for a period of time, based on the display situation of the detection line and quality control line, the sample is judged whether of containing an novel coronavirus antibody, the color contrast of the detection line is in comparison to a standard colourimetric card; if the color scale is less than 3, it is judged negative, and if greater than or equal to 3, it is judged positive.

Compared with the prior art, the present invention has the following beneficial effects: the present invention provides a lateral flow test device for detecting a coronavirus antibody by immunoassay; the test device includes three lateral flow test strips; a first test strip is directed to the detection of an antibody or antigen of a N (full-length) protein and/or an S (full-length) protein antigens/antigen; and a second test strip is directed to the antibody detection of an S-RBD-site protein antigen, and both of the first strip and the second strip are combined to detect novel coronavirus IgG and IgM antibodies, which further improves the detection ratio of the antibody by serology to practically reduce the possibility of missing detection and wrong detection, thus avoiding missing detection thoroughly; meanwhile, a third test strip is directed to the detection of a neutralizing antibody of an S-RBD site to rapidly detect the neutralizing antibody having protection effect, thus further helping the prevention of missing detection and assisting patients to perform self-detection and judge the recovery, or vaccine immunity condition, which provides more visual and reliable self-checking means for the patients to perform self-screening, control the progression and recovery of disease, or control the effect of the vaccine, and judge whether of requiring supplementary vaccination. Moreover, the test device has the following advantages of rapid detection speed and simple operation, is free from professional instrument and personnel, and easy to clinical popularization and application. Three test strips have significant complementary effects.The test device provided by the present invention is rapid, simple, reliable, prevents missing detection to the maximum extent, and requires no professional device and personnel, and is easy to clinical popularization and application. Meanwhile, the present invention further provides three groups of antigen combinations for preparing a test device for the rapid detection of novel coronavirus and uses thereof.

### Detailed Description of the Drawings

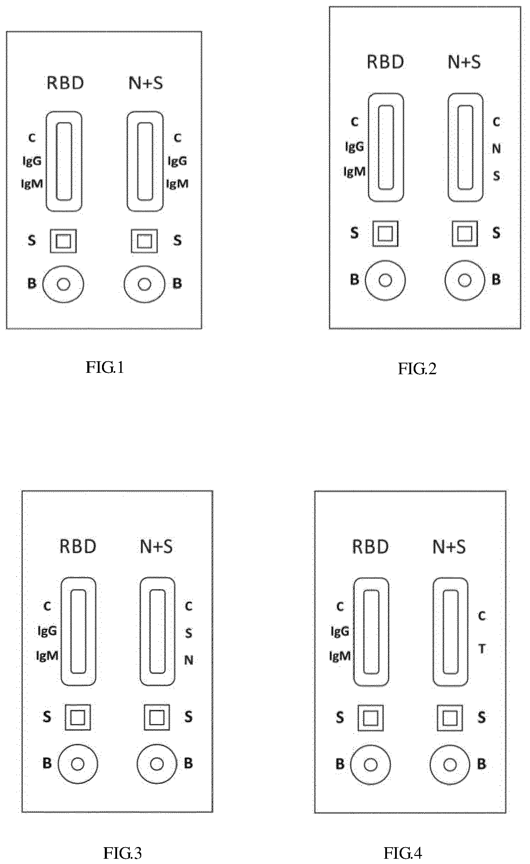

FIG. 1 is a schematic diagram of an embodiment of the present invention, where each test strip has two detection lines directed to different combinations for the detection of IgG and IgM.
FIG. 2 is a schematic diagram of a further embodiment of the present invention, where in different combinations, RBD is used to detect IgG and IgM, while for N and S, RBD is used to detect an antibody of a N antigen or an antibody of an S antigen, or N or S antigen.
FIG. 3 is a schematic diagram of a further embodiment of the present invention, where in different combinations, RBD is used to detect IgG and IgM, while for N and S, RBD is used to detect an antibody of a N antigen or an antibody of an S antigen, or N or S antigen.
FIG. 4 is a schematic diagram of a further embodiment of the present invention, where in different combinations, RBD is used to detect IgG and IgM, while for N + S , RBD is used to detect an antibody of a N antigen or an antibody of an S antigen, or N or S antigen as long as there is a N or an S corresponding antibody, or a N or an S antigen in a sample, or there is a detection line (T).

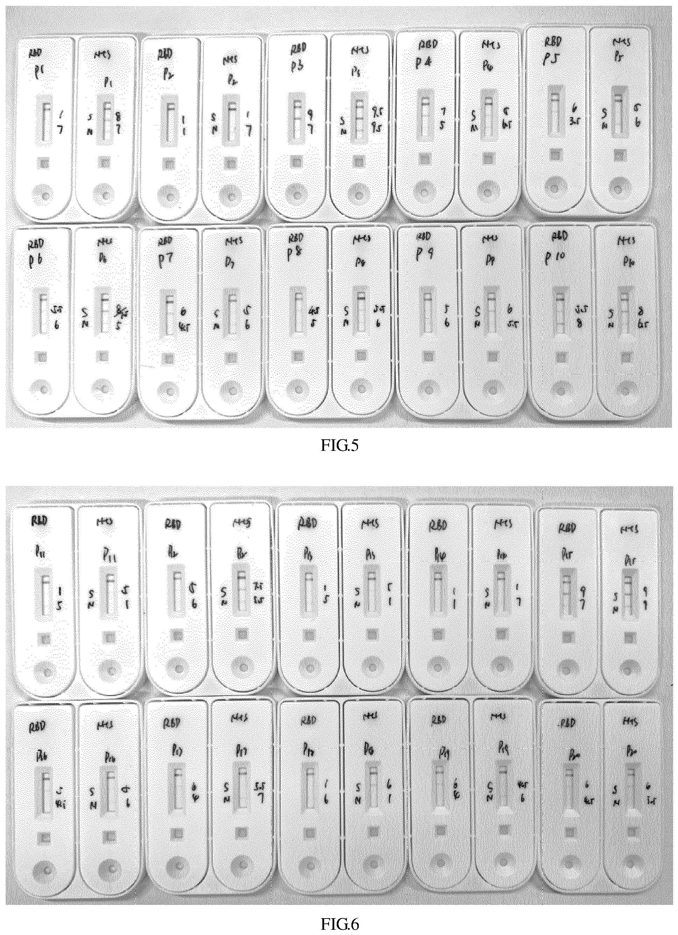

FIG. 5 is a photograph showing detection results of Example 3 of the present invention, and a schematic diagram as shown in FIG. 3 shows actual detection results (a graphical result of positive samples 1-10) (S and N antibodies are detected, S and N are separated on the same test strip, indicating that the S and N detection lines are used to detect IgG and/or IgM antibodies corresponding to S or N antigens in blood.
FIG. 6 is a photograph showing detection results of Example 3 of the present invention, and a schematic diagram as shown in FIG. 3 shows actual detection results (a graphical result of positive samples 11-20) (S and N antibodies are detected, S and N are separated on the same test strip, indicating S and N detection lines).

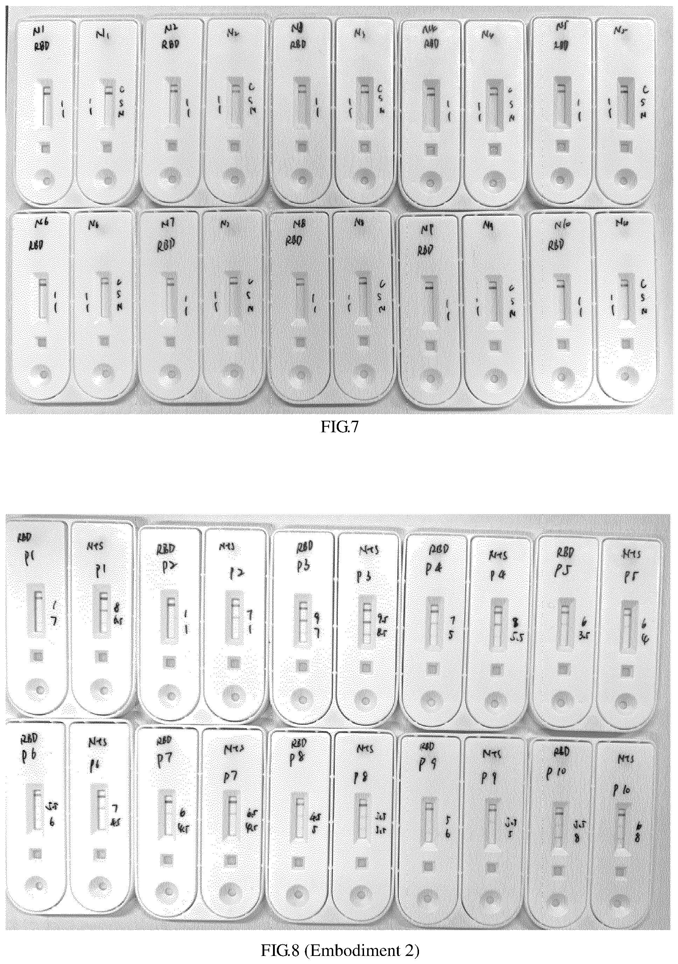

FIG. 7 is a photograph showing detection results of Example 3 of the present invention, and a schematic diagram as shown in FIG. 3 shows actual detection results (a graphical result of negative samples 1-10) (S and N antibodies are detected, S and N are separated on the same test strip, indicating that the S and N detection lines are used to detect whether there are IgG and/or IgM antibodies corresponding to S or N antigens in blood.
FIG. 8 is a photograph showing detection results of Example 2 of the present invention, and a schematic diagram as shown in FIG. 1 shows actual detection results (a graphical result of positive samples 1-10) (RBD is used to detect IgG and IgM antibodies in blood; N+S antibodies are respectively corresponding to IgG and IgM detection lines, the result is positive as long as there is any one of the two antibodies).

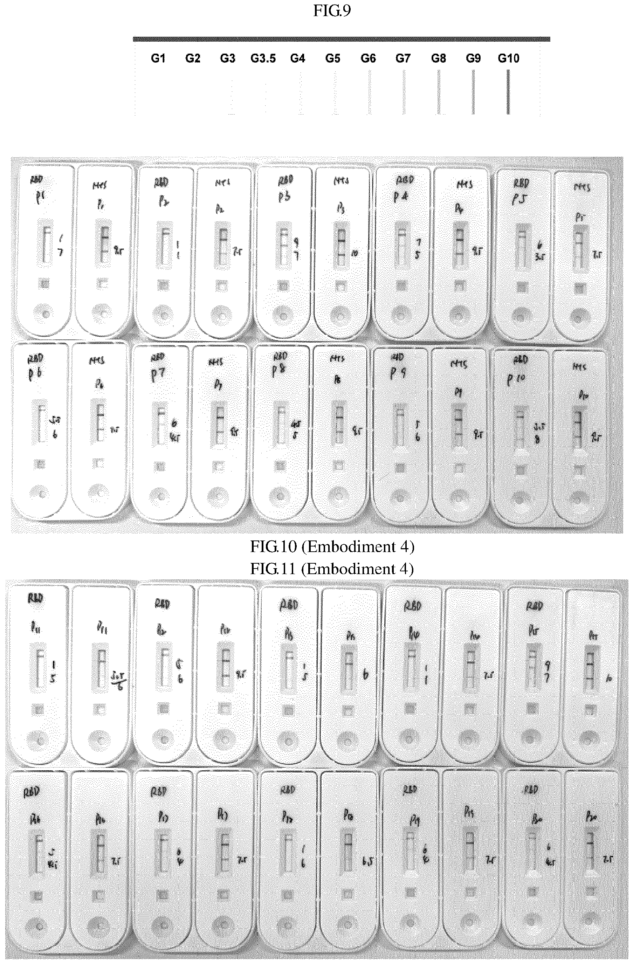

FIG. 9 is a standard colourimetric card used to judge and read the concentration or color shading on a T line.

### Detailed Description

The structure or technical terms of the present invention will be further specified below, and shall be understood and explained according to the common general terms in the art unless otherwise specified. These descriptions are only used to specify how the embodiments of the present invention are achieved by giving examples, but are not construed as limiting the present invention; and the scope of the present invention is defined and expressed by claims.

### Detection

Detection means assaying or testing whether of the presence of a substance or material. The substance or material, for example, but is not limited to chemical substances, organic compounds, inorganic compounds, metabolic products, drug or drug metabolites, organic tissues or metabolites of the organic tissues, nucleic acids, proteins or polymers. Moreover, detection further means testing the number of a test substance or material. Assay further refers to immunodetection, chemical detection, enzyme detection and the like.

### Sample

In the present invention, the sample used by the test device includes a biological liquid. The initial state of the sample may be liquid, solid or semi-solid; solid or semi-solid sample can be transformed into a liquid sample in any proper way, for example, mixing, mashing, macerating, incubating, dissolving, and enzymolysis, and then, the processed sample is poured into a collection chamber to detect whether the sample contains an analyte through a test element. The sample can be taken from human body, animals, plants, nature and the like. The sample from human body, for example, may be blood, serum, urine, cerebrospinal fluid, sweat, lymph, saliva, gastric juice and other liquid samples; faeces, hair, cutin, scale, fingernail/toenail and other solid and semi-solid samples. The sample from plants, for example, may be root, stem, leaf and other solid samples; interstitial fluid prepared by root, stem, leaf, cell sap and other liquid or semi-solid samples. The sample from the nature, for example, may be rainwater, river water, seawater, underground water and other liquid samples; soil, rock, ore, petroleum and other solid or semi-solid samples.

In some embodiments, the sample of the present invention is serum or whole blood or plasma; or during antigen detection, the sample is from a throat swab sample, a nose swab or lung sample.

### Test device

A test device generally contains a test element; the so-called test element refers to a component capable of detecting an analyte in a test sample. The detection for the analyte by the test element can be based on any technical principle, for example, immunology, chemistry, electricity, photology, molecules, physics, and the like. The test element in the present invention may be one or a combination of two or more test elements. The test element has a test area for showing detection results; after detecting, the test area shows detection results.

Various test elements can be combined together and applied to the present invention.One form is test paper.The test paper used to analyze analytes in samples (such as poison or metabolites that indicate physical conditions) can be in various forms, such as immunoassay or chemical analysis.The test paper may use an analysis mode of a non-competition law or a competition law.The test paper contains a water absorbing material having a sample adding area, a reagent area and a test area. A sample is added to the sample adding area and flows to the reagent area through a capillary action.In the reagent area, if an analyte exists, the sample is combined with an reagent.Then the sample continues to flow to the detection area.Other reagents, such as molecules that are combined with specificity of an analyte, are fixed in the detection area.These reagents react with the analyte (if existing) in the sample and the analyte is combined in the area, or the analyte is combined with a reagent in the reagent area.A mark used to display a detection signal exists in the reagent area or a separate marking area.

In the typical analysis mode of the non-competition law, if a sample includes an analyte, a signal is generated; or if the sample does not include the analyte, no signal is generated.In the competition law, if an analyte does not exist in a sample, a signal is generated; or if the analyte exists in the sample, no signal is generated.

The test element may be a test paper, and may use a water-absorbent or non-absorbent material.The test paper may include a plurality of materials for transferring a liquid sample.A material of one type of test paper can cover another material. For example, filter paper covers a nitrocellulose membrane. One area of the test paper can use one or more materials, and another area can use another or more different materials.The test paper can be stuck to a support or hard surface to improve strength of holding the test paper.

An analyte is detected by using a signal generation system. For example, a composition of one or more signal generation systems is fixed in an analyte detection area of the test paper by using one or more enzymes that specifically react with the analyte, by using the foregoing method of fixing the specificity combining substance on the test paper.A signal generation substance can be in the sample adding area, the reagent area, the detection area, or the entire test paper, and the substance can filled up one or more materials of the test paper.A solution containing the signal substance is added to a surface of the test paper or one or more materials of the test paper are immersed in a solution containing the signal substance.The test paper to which the signal containing solution is added is dried.

The various areas of the test paper can be arranged in the following manner: the sample addition area, the reagent area, the detection area, a control area, an area for determining whether the sample is adulterated, and a liquid sample absorption area.The control area is located after the detection area.All areas can be arranged on one piece of test paper using only one material.Alternatively, different areas can use different materials.Each area can come into direct contact with a liquid sample. Alternatively, different areas are arranged in a flowing direction of the liquid sample, and a tail end of each area is connected to and overlaps a front end of another area.The material used can be a material with relatively desirable water absorption, such as filter paper, a glass fiber, or a nitrocellulose membrane. The test paper may also use another form.

Generally, common reagent strips are nitrocellulose membrane reagent strips; namely, the test area includes a nitrocellulose membrane; the nitrocellulose membrane is immobilized a specific-binding molecule to show detection results; further, the reagent strips may be a cellulose acetate membrane or nylon membrane, or the like. For example, reagent strips described in some patients or devices containing the reagent strips below: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620; and US 6403383. The test strips disclosed in the above patents and similar devices containing the test strips can be applied in the test element or test device of the present invention for the detection of analytes, for example, the detection of analytes in the sample.

The test strips applied in the present invention may be the so-called lateral flow test strips; moreover, the specific structure and detection principle of these test strips are common general knowledge in the art. Common test strips include a sample collecting area, a labeled area, test area and a water absorption zone; the sample collecting area includes a sample receiving pad; the labeled area includes a label pad; the water absorption area may include an absorbent pad; and the test area includes a necessary chemical substance capable of detecting whether analytes are contained; for example, an immunoreagent or enzyme chemical reagent. A commonly used test strip is a nitrocellulose membrane reagent strips,that is, the detection area includes a nitrocellulose membrane, and a specificity combining molecule is fixed on the nitrocellulose membrane to display a detection result. The commonly used test strip may also be a cellulose acetate membrane, a nylon membrane, or the like. Certainly, a detection result control area may also be included in downstream of the detection area. Usually, the control area and the detection area appear in a form of horizontal lines, which are detection lines or control lines.Such test strip is a conventional reagent strip, and certainly, may be other type of reagent strip using capillary action for detection. In addition, the test reagent strip generally includes a dry chemical reagent component, such as a fixed antibody or another reagent. When the dry chemical reagent component encounters a liquid, the liquid flows along the reagent strip through a capillary action. As the liquid flows, the dry reagent component is dissolved in the liquid, and then proceeds to a next area to process the dry reagent in that area to react, so as to perform necessary detection. A liquid mainly flows through a capillary action.These test elements are referring to the description and records of the documents below: Study on Regeneration Treatment and Protein Adsorption Capacity of Nitrocellulose Membrane, Li Fugang; Analysis on the Chromatographic Membrane Material Property in Colloidal Gold Diagnostic Kit, Ma Hongyan, Li Qiang, *et al.,* Novel Colloidal Gold Immunochromatograohic Test Strip, Wang Yong, Wang Luhai, *et al.* The above test elements can be applied in the test device of the present invention, or configured in a test cavity in contact with a liquid sample, or used to test whether the analyte in the liquid sample entering the test cavity is present or the number thereof.

The test device herein includes two lateral flow test strips, one is used for detecting IgG and IgG antibodies in a blood sample corresponding to RBD, or used for detecting a viral antigen in a throat swab, a nose swab, lung fluid, sputum or nasal mucus, for example, a RBD antigen; or the presence of an S or a N protein antigen. Another test strip is used for detecting IgG and IgG antibodies in a blood sample corresponding to an S protein or a N protein, or used for detecting the presence of a viral antigen, namely, the S or N antigen in a throat swab, a nose swab, lung fluid, sputum or nasal mucus.

### Coronavirus

The "coronavirus" herein includes the following virus: severe acute respiratory syndrome (SARS), Middle East respiratory syndrome (MERS) and coronavirus (COVID-19). But they have certain differences in epidemiological characteristics. Globally, 10%-30% of upper respiratory tract infections are caused by four types of coronaviruses: HCoV-229E, HCoV-OC43, HCoV-NL63, and HCoV-HKU1, which are the second cause of the common cold, second only to the nose virus.The infection shows seasonal epidemic, with high incidence of the disease in each spring and winter. The incubation period is 2-5 days, and the population is generally susceptible, mainly transmitting through human-to-human contact.

SARS is caused by human infection with SARS-CoV, which first appears in parts of Guangdong Province in China, and then spread to 24 provinces, autonomous regions and municipalities in China and 28 other countries and regions around the world.During the first SARS epidemic in the world from November 2002 to July 2003, a total of 8096 cases diagnosed clinically and 774 deaths were reported globally, with a mortality of 9.6%.The incubation period of SARS is usually limited to 2 weeks, usually about 2 to 10 days.The population is generally susceptible to SARS, the main source of infection of which is SARS patients, especially patients with obvious symptoms that are highly infectious, while patients in the incubation period or cured are not infectious.

MERS is a viral respiratory disease caused by MERS-CoV, which is first confirmed in Saudi Arabia in 2012.Since 2012, MERS has spread to 27 countries and regions such as the Middle East, Asia, Europe, etc., and 80% of cases are from Saudi Arabia, with a mortality of about 35%.The incubation period is up to 14 days, and the population is generally susceptible.Dromedary camel is a major host of MERS-CoV and is the main source of infections across humans, with limited human-to-human transmission.

In 2019, a serious cold (COVID-19) caused by the coronavirus broke out in Wuhan. As of March 10, 2020, the cumulative number of infections in China exceeded 80,000, with a severe illness rate of about 18.5% and a mortality of about 2%, meanwhile, COVID-19 was in an outbreak period.The high infectiousness, high pathogenicity, high rate of severe illness and high mortality of such coronavirus have caused major harm to the global economy, society, and health.

### Detailed Description of the Embodiments

The present invention is further described in detail in combination with the examples below; and it should be indicated that the examples below aim at understanding the present invention conveniently, and are not construed as limiting the present invention. Reagents not pointed out specifically in the example are products known to a person skilled in the art, and are purchased from products available in the market.

### Example 1 Preparation of a lateral flow test device for detecting a novel coronavirus antibody by immunoassay provided by the prevent invention

The lateral flow test device for detecting a coronavirus antibody by immunoassay prepared by the embodiment includes a test strip 1, test strip 2 and a test strip 3; the test strip 1, test strip 2 and the test strip 3 have a roughly same structure, namely, according to a direction of liquid flow, there are successively a sample area including sample loading wells S and buffer solution wells B which are located on the sample area, a label area, a detection area, and a water absorbing area from upstream to downstream; a common water-absorbing filter paper is used as a water absorbing pad for preparation in the water absorbing area; a sample applying pad is used in the sample area, and is made of glass fiber; samples added via sample loading wells S flow onto the glass fiber, and then the buffer solution added via B holes flow onto the glass fiber, and then are mixed with samples to flow onto the label pad; the label area is prepared into a label pad, including an antigen or antibody coupled by a labeled particle (such as, a gold particle, a latex particle or a dye, or other colored mark); afterwards, a spray device is used to spray the labeled mixture on a polyester film to be prepared into a label pad, and the mark on the label pad can flow with liquid; a nitrocellulose membrane is used in a detection area; a buffer solution PBS is used to dissolve the antibody or antigen on the detection line; then a membrane-dotting device is used to line out on the nitrocellulose membrane to keep the distance among different antibodies within 3-8 mm, then the nitrocellulose membrane is placed in an oven for drying for further use; further, the antibody, antigen or other binding substances treated on the membrane are generally unmovable.

The water absorbing area, sample area, label area, and the detection area are assembled after preparation; one end of the sample applying pad is overlapped on one end of the label pad, and another end of the label pad is overlapped on the nitrocellulose membrane; the nitrocellulose membrane on one end of the quality control line is overlapped by the water-absorbing filter paper to form an entire test strip, and then, the test strip is assembled in a detection card, of which sample loading wells S and buffer solution wells B on the detection card are corresponding to the sample applying pad, and the nitrocellulose membrane corresponds to a degree window; and the sample loading wells are located downstream of the buffer solution wells B.

Example 2: an S full-length protein antigen and a N full-length protein antigen were coated in the label area of a first test strip; a RBD antigen was coated in a label area of a second test strip; a colloidal gold-labeled S-RBD antigen was coated on a label pad of a third test strip; and an angiotensin converting enzyme 2 (ACE2) was coated or immobilized on a detection line.

The method for preparing a lateral flow test device for detecting a novel coronavirus antibody by immunoassay in this example is with reference to the way of Example 1 except that reagents on the label pad and detection line are different.

To the first test strip: a goat anti-chicken IgY antibody was coated or immobilized on a quality control line of a nitrocellulose membrane, a rat anti-human IgG antibody was coated or immobilized on an IgG detection line; a rat anti-human IgM antibody was coated or immobilized on an IgM detection line; and a label pad is coated with a colloidal gold-labeled S full-length protein and N full-length protein (antigen) and colloidal gold-labeled chicken-IgY antibody (used for the quality control line). During the treatment of the marker, the mass ratio of the S full-length protein to the N full-length protein is 20:1-5:1. The mass ratio of the S full-length protein to the N full-length protein is 20:1 herein (right test strip in the schematic diagram of FIG.1).

To the second test strip: a goat anti-chicken IgY antibody was coated or immobilized on a quality control line of a nitrocellulose membrane, a rat anti-human IgG antibody was coated or immobilized on an IgG detection line; a rat anti-human IgM antibody was coated on an IgM detection line; and a label pad is coated with a colloidal gold-labeled RBD antigen and a gold-labeled chicken-IgY antibody. IgG detection line was used for detecting IgG in a sample; IgM detection line was used for detecting IgM in a sample; the treatment was performed according to the treatment way of the detection area in Example 1 (left test strip in the schematic diagram of FIG.1).

To the third test strip: an angiotensin converting enzyme 2 (ACE2) was coated or immobilized on a detection line of a nitrocellulose membrane; and a label pad was coated with a gold-labeled S-RBD antibody (antigen).

Detection principle of the first test strip: if there was an antibody (IgG or IgM) of the S or N full-length protein (antigen), the antigen on a label pad would bind the antibody in a sample to form: metal particle-S or Nfull-length protein (antigen)-IgG or IgM (sample); then the obtained product was captured by a human IgG antibody immobilized on a detection line, or a human IgM antibody to obtain a particle complex, thus forming a positive or negative result (metal particle-S protein or N protein (antigen)-IgG or IgM (sample)-human IgG antibody, or human IgM antibody) (an indirect method). N or S protein can be detected as long as one or two of them produced antibodies in a human body due to infection.

Detection principle of the second test strip: if there was an antibody (IgG or IgM) of the S protein-RBD (identifying area), the antigen on a label pad would bind the antibody in a sample to form: metal particle-S protein-RBD-IgG or IgM; then the obtained product was captured by a human IgG antibody immobilized on a detection line, or a human IgM antibody to obtain a particle complex, thus forming a positive or negative result (metal particle-S protein-RBD (antigen)-IgG or IgM (sample)-human IgG antibody, or human IgM antibody).

Detection principle of the third test strip: if there was novel coronavirus neutralizing antibody in a blood sample, the antigen on a label pad would bind the antibody in a sample to form: metal particle-S-RBD protein (antigen)-neutralizing antibody (sample), so that ACE2 on the detection line cannot bind the S-RBD protein, thus forming a positive result (metal particle-S-RBD protein (antigen)-neutralizing antibody (sample)) (a competition method). If there was no novel coronavirus neutralizing antibody in the blood sample, the antigen on a label pad was in free state, so that ACE2 on the detection line bonded the S-RBD protein, thus forming colored strips and showing a negative result (metal particle-S-RBD protein (antigen)-ACE2) (a competition method).

The first test strip, second test strip and the third test strip were respectively placed on a detection card, and a positive blood sample or negative blood sample was dropwisely added to sample applying wells (square wells) simultaneously, then a buffer solution (round holes) was dropwisely added to another well ; the buffer solution was a phosphate buffer with PH=7.4. 20 positive samples P1-P20 (these positive samples were confirmed clinically: the sample was that a throat swab sample contained coronavirus after through nucleic acid testing) and 20 negative samples N1-N20 (samples confirmed clinically: the sample was a negative sample of not containing coronavirus after through nucleic acid testing of a throat sample (throat swab)) were detected to obtain the detection results as shown in Table 1:

**Table 1: detection results of Example 2**

| No. | (N+S) (reading of the first test strip) | | (RBD) (reading of the second test strip) | | (S-RBD) (reading of the third test strip) |
|---|---|---|---|---|---|
| | IgG | IgM | IgG | IgM | A competition law |
| P1 | 8. | 6.5. | 1. | 7. | G2 |
| P2 | 7. | 1. | 1. | 1. | G2 |
| P3 | 9.5. | 8.5. | 9. | 7. | G2 |
| P4 | 8. | 5.5. | 7. | 5. | G2 |
| P5 | 6. | 3.5. | 6. | 3.5. | G1 |
| P6 | 7. | 4.5. | 5.5. | 6. | G2 |
| P7 | 6. | 4.5. | 6. | 4.5. | G2 |
| P8 | 4.5. | 5. | 4.5. | 5. | G2 |
| P9 | 5. | 6. | 5. | 6. | G2 |
| P10 | 6. | 8. | 5.5. | 8.5. | G2 |
| P11 | 1. | 5. | 1. | 5. | G2 |
| P12 | 7. | 5. | 5. | 6. | G4 |
| P13 | 1. | 5.5. | 1. | 5. | G2 |
| P14 | 7. | 1. | 4. | 1. | G2 |
| P15 | 9. | 8. | 9. | 7. | G1 |
| P16 | 6. | 4. | 5. | 4.5. | G2 |
| P17 | 7. | 5. | 6. | 4. | G2 |
| P18 | 1. | 6. | 1. | 6. | G3 |
| P19 | 6. | 4.5. | 6. | 4. | G2 |
| P20 | 6. | 4.5. | 6. | 4.5. | G2 |
| N1 | 1. | 1. | 1. | 1. | G9 |
| N2 | 1. | 1. | 1. | 1. | G9 |
| N3 | 1. | 1. | 1. | 1. | G9 |
| N4 | 1. | 1. | 1. | 1. | G9 |
| N5 | 1. | 1. | 1. | 1. | G9 |
| N6 | 1. | 1. | 1. | 1. | G9 |
| N7 | 1. | 1. | 1. | 1. | G9 |
| N8 | 1. | 1. | 1. | 1. | G9 |
| N9 | 1. | 1. | 1. | 1. | G9 |
| N10 | 1. | 1. | 1. | 1. | G9 |
| N11 | 1. | 1. | 1. | 1. | G9 |
| N12 | 1. | 1. | 1. | 1. | G9 |
| N13 | 1. | 1. | 1. | 1. | G9 |
| N14 | 1. | 1. | 1. | 1. | G9 |
| N15 | 1. | 1. | 1. | 1. | G9 |
| N16 | 1. | 1. | 1. | 1. | G9 |
| N17 | 1. | 1. | 1. | 1. | G9 |
| N18 | 1. | 1. | 1. | 1. | G9 |
| N19 | 1. | 1. | 1. | 1. | G9 |
| N20 | 1. | 1. | 1. | 1. | G9 |

The color concentration or shading of the detection lines on the first test strip and the second test strip was in comparison to a standard colourimetric card (FIG. 9) (the colourimetric card shows the concentrations of the positive structure line at different concentrations, and has 1-10 color depth to form a gradient); if the color scale was judged 3 below, it was judged negative; if greater than or equal to 3, it was judged positive. Specific results were referring to partial positive results in FIG. 8; there was no negative result.

The color concentration or shading of the detection line on the third test strip was in comparison to a standard colourimetric card (the colourimetric card shows the concentrations of the positive structure line at different concentrations, and has G1-G10 color depth to form a gradient); if the color scale of the detection line was less than or equal to 3 (G3), it was judged positive; if greater than 3 (G3), it was judged negative. Specific results were partial positive results; there was no negative result.

It can be seen from Table 1 that for negative samples N1-N20, the reading of the first test strip and the second test strip was 1, indicating negative, which was consistent with the actual sample and the detection result of nucleic acid testing. As for positive samples P1-P20, if the RBD antigen labeled by colored particles (test strip 2) was used for detection, there were 5 negative results (P1, P2, P11, P13 and P18) in 5 samples recognized as positive for IgG; in the test results of N+S (test strip 1), there were three negative results (P11, P13 and P18) in 5 positive samples for IgG; other samples were positive (P1, P2). It indicates that N+S detection may at least detect 2 samples positive more. IgG is an antibody which is present after infection or recovery, indicating that IgG antibodies produced by RBD were detected, there is a phenomenon of missing detection possibly. This was probably because the nucleic acid positive sample was a throat swab sample, while a blood sample was used in the prevent invention; generally, blood produces antibodies several days after infection, for example, the earliest is IgM, and then IgG is produced secondly usually. If antibodies are detected only by a RMD antigen, it may cause IgG missing detection, which may show that in the sample, IgM is positive, but IgG is negative, thereby resulting in improper or wrong therapeutic measures.

In combination with the result of IgM below, for a P2 sample, if a RBD antigen is used for antibody detection, both IgM and IgG are negative, which may indicate that the patient is negative, so that the patient will be not further detected via nucleic acid confirmation, resulting in missing detection, and the consequence is to cause a wider range of infectivity, or infect healthy people. On the contrary, if N+S way is used for supplementary detection, the P2 sample is positive, and at least IgG is detected positive; even though IgG means a sign of patients' recovery or means that patients have been infected, the treatment strategy or medical strategy or protection strategy can be at least changed, for example, nucleic acid is confirmed to detect where the virus is present in a body.

For a P1 sample, N+S combined detection shows that IgM and IgG are positive; if RBD is used for detection alone, IgM is positive and IgG is negative, which at least indicates that single RBD detection is not comprehensive and cannot achieve a comprehensive assessment. N+S can comprehensively assess the infection status of a patient.

For IgM, there is a same result to the RBD antigen detection and N+S antigen detection; namely, both are negative (P2 and P14), but the reading measured has a difference; test strip 1 is used in some samples, the detection result is higher (such as, P3, P4, P13, and P15); and test strip 2 is used in some samples, the detection result is higher (such as, P1, P6, P10, P16). It indicates that in some samples, the concentration of total antibodies of N+S in blood is higher, while the concentration of RBD antibodies is low, or for some samples, the concentration of RBD antibodies is high, while the concentration of N+S antibodies is low. It may at least indicate that when some samples are in a uncertain state, for example, within a color reading range from 3.0 to 3.5, if only one index is used at this time, for example, only RBD, or N or S is used for detection, the result is uncertain. Moreover, the combined detection may further confirm the reliability of the detection results from each different test results, thus avoiding missing detection, or avoiding false negative or false positive, especially, false negative.

For example, when a similar P17 sample was detected by RBD, there were results of 6 (IgG) and 4 (IgM); while when it was detected by N+S, there were results of 7 (IgG) and 5 (IgM), that is, there was just an order of color difference. In case of a similar sample, when the content of antibodies in the sample or the binding capacity of antibodies to antigens was lower than that in P17 sample, and RBD detection was performed at this time, there were possibly results of 5 (IgG)and 3 (IgM), and thereby IgM may be regarded as negative. But N+S was used for supplementary detection, the interpretation result may be 5 (IgG) and 4 (IgM), and thereby IgM may be regarded as positive. There was further a slightly extreme case, when a single RBD was used for detection, there were results of 2 (IgG) and 2 (IgM), and thereby the sample may be regarded as negative at this time; but when N+S was used for supplementary detection, and if there were results of 3 (IgG) and 3 (IgM), a patient of the sample may be regarded to be or very likely to be infected virus, which may at least remind the detector that the patient of the sample needed to be further confirmed, for example, confirmation of the detection via nucleic acid testing.

This is because it is inclined that the patient has been infected novel coronavirus and in acute infection period in the route of infection and transmission of the virus, and needs to be further confirmed or cubicle treatment, thus rendering the original negative result to be confirmed positive more. Certainly, the patient may be subjected to the nucleic acid testing of a throat swab or a blood sample, thus further confirming the detection. Some virus carriers show no symptom, but are infectious. In this way, the detector can obtain accurate detection results as much as possible, thus taking improper measures to make further treatment and preventive measures.

To sum up, during the detection of IgG and IgM at the same time, the second test strip will cause missing detection of P2 and P14; if the first test strip is used for supplementary detection, the missing detection is avoided completely; for the detection of IgG, if the first test strip and the second test strip are combined for test, it may avoid the missing detection of IgG antibodies, and improve comprehensive detection to prevent missing detection; for the detection of IgM, even though the first test strip and the second test strip have completely detected the IgM positive results, resulting in not obvious effect of missing detection, it cannot be denied that the first test strip and the second test strip have supplementary effect, in case of a weakly positive sample (the content of IgM antibodies is lower), the complementary detection of the first test strip and the second test strip will obviously prevent missing detection via reading differences. In case of the detection by RBD antibodies only, the probability of negative results will be very high; and in case of supplementary detection by N+S, the result will be positive to make up for the natural defect detected by RBD antibodies (RBD shows a weakly positive result to some samples).

After through a large amount of experiments, in case of the detection by single RBD, 50 blood samples were randomly selected for detection, and 5 samples showed weakly positive (directed to IgG and IgM); and the reading was about 3 or close to 3; while in case of the detection by N+S, all the results were positive, and the reading results were within 4-5. Patients of the 5 samples received nucleic acid testing via throat swab samples and confirmed as positive. This further confirms that single/combined detection may have beneficial supplementary confirmation.

It can be seen from Table 1 that for negative samples N1-N20, the reading of the third test strip was G9, indicating negative, which was consistent with the actual sample; and the result displayed negative, indicating that the concentration of neutralizing antibodies was lower than the effective protection scope.

For positive samples P1-P20, most of the readings on the test strips were G2, namely, positive, indicating that the concentration of neutralizing antibodies was within the effective protection scope; the reading of P15 was G1, indicating that the content of neutralizing antibodies was higher, and a patient may have stronger autoimmunity; the reading of P18 was G3, indicating that the content of neutralizing antibodies was lower; the reading of P12 was G4, indicating that the patient was infected with novel coronavirus and the antibody was positive, but the content of neutralizing antibodies may be negative, which was judged early infection, and there was no enough neutralizing antibodies capable of resisting the virus, and at this time, it was recommended to take measure in time to help patients enhancing disease resistance.

Moreover, by making a comparison to the detection results between the first and second test strips of P2, it can be seen that there is a possibility of missing detection in case of the detection by the second test strip; and the third test strip may be used to help avoiding missing detection; meanwhile, the third test strip can further provide the detection results of neutralizing antibodies, so that the patient can judge the progression of disease.

### Example 3: an S full-length protein and a N full-length protein were coated in a label area of a first test strip; a RBD antigen was coated in a label area of a second test strip; a colloidal gold-labeled S-RBD antigen was coated on a detection line of a third test strip; and an angiotensin converting enzyme 2 (ACE2) was coated on a detection line.

The method for preparing a lateral flow test device for detecting a novel coronavirus antibody by immunoassay in this example is with reference to the way of Example 1.

A goat anti-mouse IgY antibody was coated or immobilized on a quality control line of a nitrocellulose membrane; a N full-length protein (antigen) was coated on a N detection line; an S full-length protein (antigen) was coated on an S detection line; a label pad is coated with a colloidal gold-labeled RBD antigen and a colloidal gold-labeled rat anti-human IgG antibody and rat anti-human IgM antibody (excessive). What was detected on the N detection line of the test strip 1 was the antibody of the N full-length protein antigen (including IgG and IgM); What was detected on the S detection line was the antibody of the S full-length protein antigen (including IgG and IgM). That is, for N or S antigen, as long as the sample contained IgG or IgM of the N antigen; or IgG or IgM of the S antigen, it showed a positive result on the N detection line, otherwise, it was a negative result.

To the second test strip: a goat anti-chicken IgY antibody was coated on a quality control line of a nitrocellulose membrane, a rat anti-human IgG antibody was coated or immobilized on an IgG detection line; a rat anti-human IgM antibody was coated on an IgM detection line; and a label pad is coated with a colloidal gold-labeled RBD antigen and a gold-labeled chicken-IgY antibody.IgG detection line on the test strip 2 was used for detecting IgG; and the IgM detection line was used for detecting IgM.

The detection line on a nitrocellulose membrane of the third test strip was coated with a **colloidal gold-labeled S-RBD (antigen); and an angiotensin converting enzyme 2 (ACE2) was coated on a detection line.**

Detection principle of the first test strip: if there was an antibody (IgG or IgM) of the S or N full-length protein (antigen) in a blood sample, the antigen on a label pad would bind the antibody in a sample to form: metal particle-mouse anti-human IgG antibody or rat anti-human IgM antibody-IgG or IgM (sample); then the obtained product was captured by the N or S full-length protein on the detection line to obtain a particle complex, thus forming a positive or negative result (metal particle-mouse anti-human IgG antibody or rat anti-human IgM antibody-IgG or IgM (sample)-S protein or N full-length protein.

Detection principle of the second test strip: if there was an antibody (IgG or IgM) of the S protein-RBD in a blood sample, the antigen on a label pad would bind the antibody in a sample to form: metal particle-S protein-RBD-IgG or IgM; then the obtained product was captured by a human IgG antibody immobilized on a detection line, or a human IgM antibody to obtain a particle complex, thus forming a positive or negative result (metal particle-S protein-RBD (antigen)-IgG or IgM (sample)-human IgG antibody, or human IgM antibody).

Detection principle of the third test strip: if there was novel coronavirus neutralizing antibody in a blood sample, the antigen on a label pad would bind the antibody in the sample to form: ACE2-neutralizing antibody (sample), so that the S-RBD protein on the detection line cannot bind ACE2, thus forming a positive result (ACE2-neutralizing antibody (sample)-metal particle-S-RBD protein (antigen) (a competition method). If there was no novel coronavirus neutralizing antibody in the blood sample, ACE2 on a label pad was in free state, so that S-RBD protein (antigen) on the detection line bonded ACE2, thus forming colored strips and showing a negative result (metal particle-S-RBD protein (antigen)-ACE2) (a competition method).

The first test strip, second test strip and the third test strip were respectively placed on a detection card, and a positive blood sample was dropwisely added to sample applying wells simultaneously, then a buffer solution was dropwisely added to another well; the buffer solution was a phosphate buffer with PH=7.4.20 positive samples P1-P20 (these positive samples confirmed clinically) and 20 negative samples N1-N20 (samples confirmed clinically) were detected to obtain the detection results as shown in Table 2:

**Table 2: detection results of Example 3**

| No. | (N+S) | | (RBD) | | (S-RBD) (a reading of the third test strip) |
|---|---|---|---|---|---|
| | N | S | IgG | IgM | A competition law |
| P1 | 7. | 8. | 1. | 7. | G2 |
| P2 | 7. | 1. | 1. | 1. | G2 |
| P3 | 10. | 9. | 9. | 7. | G3 |
| P4 | 7. | 5. | 7. | 5. | G2 |
| P5 | 6. | 5. | 6. | 3.5. | G2 |
| P6 | 5. | 8. | 5.5. | 6. | G2 |
| P7 | 6. | 5. | 6. | 4.5. | G2 |
| P8 | 6.5. | 5.5. | 4.5. | 5. | G2 |
| P9 | 5. | 5.5. | 5. | 6. | G2 |
| P10 | 6.5. | 8. | 5.5. | 8.5. | G2 |
| P11 | 1. | 5. | 1. | 5. | G2 |
| P12 | 5. | 8. | 5. | 6. | G4 |
| P13 | 1. | 5. | 1. | 5. | G2 |
| P14 | 7. | 1. | 4. | 1. | G2 |
| P15 | 9.5. | 9. | 9. | 7. | G1 |
| P16 | 6. | 5. | 5. | 4.5. | G2 |
| P17 | 7. | 5.5. | 6. | 4. | G2 |
| P18 | 1. | 6. | 1. | 6. | G3 |
| P19 | 6. | 4.5. | 6. | 4. | G2 |
| P20 | 5.5. | 6. | 6. | 4.5. | G2 |
| N1 | 1. | 1. | 1. | 1. | G9 |
| N2 | 1. | 1. | 1. | 1. | G9 |
| N3 | 1. | 1. | 1. | 1. | G9 |
| N4 | 1. | 1. | 1. | 1. | G9 |
| N5 | 1. | 1. | 1. | 1. | G9 |
| N6 | 1. | 1. | 1. | 1. | G9 |
| N7 | 1. | 1. | 1. | 1. | G9 |
| N8 | 1. | 1. | 1. | 1. | G9 |
| N9 | 1. | 1. | 1. | 1. | G9 |
| N10 | 1. | 1. | 1. | 1. | G9 |
| N11 | 1. | 1. | 1. | 1. | G9 |
| N12 | 1. | 1. | 1. | 1. | G9 |
| N13 | 1. | 1. | 1. | 1. | G9 |
| N14 | 1. | 1. | 1. | 1. | G9 |
| N15 | 1. | 1. | 1. | 1. | G9 |
| N16 | 1. | 1. | 1. | 1. | G9 |
| N17 | 1. | 1. | 1. | 1. | G9 |
| N18 | 1. | 1. | 1. | 1. | G9 |
| N19 | 1. | 1. | 1. | 1. | G9 |
| N20 | 1. | 1. | 1. | 1. | G9 |

The color of the detection line is in comparison with a standard colourimetric card; if the color scale is less than 3, it is judged negative, and if greater than or equal to 3, it is judged positive.

It can be seen from Table 2 that for negative samples N1-N20, the reading of the first test strip and the second test strip was 1, indicating negative, which was consistent with the actual sample.

For positive samples P1-P20, the second test strip will cause missing detection to P2; but the N protein antigen in the first test strip may supplement the effect to prevent missing detection; if the N protein antigen was detected separately, it may cause missing detection of 3 samples (P11, P13, and P19); at this time, the S protein antibody or the RBD antibody on the second test strip detected IgM to provide a supplementary effect to prevent missing detection; if the S protein antibody was detected separately, it may cause missing detection of 2 samples (P2, P14), and at this time, the N protein antibody or the RBD antibody on the second test strip was required to detect IgG to provide a supplementary effect, thus preventing missing detection. It can be seen that the N protein, S protein on the first test strip, and the RBD protein on the second test strip have supplementary effect to increase of the possibility of effectively reducing missing detection, which accords with the actual situation. In other words, only RBD antibody is used for detection, resulting in missing detection to a P2 sample; when N+S combination way is used for detection, even though some samples will be detected negative only via N, or will be detected negative only via S. The N+S combination way (the detection ratio of each N and S is not distinguished separately), has 100% positive detection rate, which is consistent with the positive result. Generally, nucleic acid testing is a gold standard to the detection novel coronavirus as long as a blood sample or throat swab sample contains novel coronavirus which can be confirmed by nucleic acid testing. The antibody detection is performed within a certain period of time after virus infection, for example, IgM antibodies appear within 5-7 d, and then IgG antibodies appear within 10-15 d. But the production of an antibody has hysteretic nature. If samples are taken more than 5 days, the antibody may be detected negative, if RBD is used for detection only, there is surely missing detection; while N+S is used for detection, the result is 100% consistent with the nucleic acid testing.

It can be seen from Table 2 that for negative samples N1-N20, the reading of the third test strip was G9, indicating negative, which was consistent with the actual sample; and the result displayed negative, indicating that the concentration of neutralizing antibodies was lower than the effective protection scope.

For positive samples P1-P20, most of the readings on the test strips were G2, namely, positive, indicating that the concentration of neutralizing antibodies was within the effective protection scope; the reading of P15 was G1, indicating that the content of neutralizing antibodies was higher; even though there was a higher amount of viral antigens in the body, the patient had stronger autoimmunity to produce enough neutralizing antibodies to resist the virus, indicating that the patient was in a process of gradual recovery; the reading of P12 was G4, indicating that the patient was infected with novel coronavirus and the antibody was positive, but the content of neutralizing antibodies may be negative, which was judged early infection, and there was no enough neutralizing antibodies capable of resisting the virus, and at this time, it was recommended to take measures in time to help patients enhancing disease resistance.

Similarly, by making a comparison to the detection results between the first and second test strips of P2, it can be seen that there is a possibility of missing detection in case of the detection by the second test strip; and the third test strip may be used to help avoiding missing detection; meanwhile, the third test strip can further provide the detection results of neutralizing antibodies, so that the patient can judge the progression of disease.

### Example 4: detection per day at regular intervals

The method for preparing a lateral flow test device for detecting a novel coronavirus antibody by immunoassay in this example is with reference to the way of Example 2.An S full-length protein antigen and a N full-length protein antigen were coated in the label area of a first test strip; a RBD antigen was coated in a label area of a second test strip; a colloidal gold-labeled S-RBD antigen was coated on a label pad of a third test strip; and an angiotensin converting enzyme 2 (ACE2) was coated or immobilized on a detection line.

The first test strip, second test strip and the third test strip were respectively placed on a detection card, and a positive blood sample or negative blood sample was dropwisely added to sample applying wells (square wells) simultaneously, then a buffer solution (round holes) was dropwisely added to another well ; the buffer solution was a phosphate buffer with PH=7.4.Blood samples of each patient were detected at regular intervals to obtain the detection results as shown in Table 3:

**Table 3: detection results of Example 4**

| No. | (N+S) (reading of the first test strip) | | (RBD) (reading of the second test strip) | | (S-RBD) (a reading of the third test strip) |
|---|---|---|---|---|---|
| | IgG | IgM | IgG | IgM | A competition law |
| Day 1 | 1. | 1. | 1. | 1. | G9 |
| Day 2 | 1. | 3.5. | 1. | 3. | G9 |
| Day 3 | 1. | 5. | 1. | 4.5. | G9 |
| Day 4 | 1. | 7. | 1. | 7.5. | G5 |
| Day 5 | 3.0. | 9. | 6. | 9.5. | G5 |
| Day 6 | 5.5. | 8. | 5.5. | 8.5. | G4 |
| Day 7 | 6. | 8. | 6. | 8. | G3 |
| Day 8 | 7.5. | 7. | 7.5. | 7.5. | G3 |
| Day 9 | 8. | 6. | 8. | 6. | G2 |
| Day 10 | 6.5. | 5.5. | 7.5. | 5. | G2 |
| Day 11 | 5.5. | 4.5. | 5.5. | 6. | G2 |
| Day 12 | 6. | 4.5. | 6. | 4.5. | G2 |
| Day 13 | 5. | 4. | 5. | 4.5. | G1 |
| Day 14 | 5. | 4. | 5. | 4. | G2 |
| Day 15 | 6. | 4.5. | 6. | 4.5. | G2 |
| Day 16 | 5. | 4.5. | 5.5. | 4.5. | G2 |
| Day 17 | 5. | 4.5. | 5. | 4.5. | G2 |
| Day 18 | 4.5. | 4. | 4.5. | 4. | G2 |
| Day 19 | 5. | 4.5. | 5. | 4.5. | G2 |
| Day 20 | 5.5. | 4. | 4.5. | 4. | G1 |
| Day 21 | 4. | 4.5. | 4.5. | 4. | G2 |
| Day 22 | 6. | 4.5. | 6. | 4.5. | G2 |
| Day 23 | 4.5. | 4. | 4.5. | 4. | G2 |
| Day 24 | 5. | 4.5. | 5. | 3. | G2 |
| Day 25 | 6. | 3.5. | 6. | 3.5. | G2 |
| Day 26 | 5. | 3. | 5.5. | 2.5. | G2 |
| Day 27 | 4.5. | 3. | 4.5. | 2. | G2 |
| Day 28 | 4.5. | 2.5. | 4.5. | 2.5. | G2 |
| Day 29 | 3. | 2.5. | 3. | 2. | G2 |
| Day 30 | 3. | 2. | 2.5. | 1.5. | G2 |

It can be seen from Table 3 that the initial stage of infection was on the earlier 6 days; and the IgM and IgG antibodies were also gradually detected, but there was no effective neutralizing antibody; starting from the 7th day, the neutralizing antibody can be detected via a third test strip, indicating that the patient was generating resistance to the virus; from days 5 to 9, the total amount of antibodies produced can be detected to be up to a peak; from the 9th day, the content of the own neutralizing antibodies had been up a higher level, and the body started to enter a state of recovery till the 30th day, the patient's body always kept a higher level of neutralizing antibodies,being G2 around; but the total amount of antibodies had declined gradually, indicating that the patient would be recovery, and had certain resistance to the virus.

### Example 5:

A RBD antigen was coated on a label area of a second test strip, and there was only one detection line in a detection area of the first test strip; and an antibody of a N or an S protein antigen was immobilized thereon. The specific treatment differed from the first test strip in Example 3 in that N and S were not distinguished, and mixedly treated in the label area; and the same sample was taken for detection to obtain the following detection results.

**Table 4: detection results of Example 5**

| No. | (RBD) | | (N+S) | (S-RBD) (a reading of the third test strip) |
|---|---|---|---|---|
| | IgG | IgM | T | A competition law |
| P1 | 1. | 7. | 9.5. | G2 |
| P2 | 1. | 1. | 7.5. | G2 |
| P3 | 9. | 7. | 10. | G2 |
| P4 | 7. | 5. | 9.5. | G2 |
| P5 | 6. | 3.5. | 7.5. | G2 |
| P6 | 5.5. | 6. | 9.5. | G2 |
| P7 | 6. | 4.5. | 9.5. | G2 |
| P8 | 4.5. | 5. | 9.5. | G2 |
| P9 | 5. | 6. | 9.5. | G2 |
| P10 | 5.5. | 8.5. | 9.5. | G2 |
| P11 | 1. | 5. | 6. | G2 |
| P12 | 5. | 6. | 9.5. | G4 |
| P13 | 1. | 5. | 6. | G2 |
| P14 | 1. | 1. | 7.5. | G2 |
| P15 | 9. | 7. | 10. | G1 |
| P16 | 5. | 4.5. | 7.5. | G2 |
| P17 | 6. | 4. | 7.5. | G2 |
| P18 | 1. | 6. | 6.5. | G3 |
| P19 | 6. | 4. | 7.5. | G2 |
| P20 | 6. | 4.5. | 7.5. | G2 |
| N1 | 1. | 1. | 1. | G9 |
| N2 | 1. | 1. | 1. | G9 |
| N3 | 1. | 1. | 1. | G9 |
| N4 | 1. | 1. | 1. | G9 |
| N5 | 1. | 1. | 1. | G9 |
| N6 | 1. | 1. | 1. | G9 |
| N7 | 1. | 1. | 1. | G9 |
| N8 | 1. | 1. | 1. | G9 |
| N9 | 1. | 1. | 1. | G9 |
| N10 | 1. | 1. | 1. | G9 |
| N11 | 1. | 1. | 1. | G9 |
| N12 | 1. | 1. | 1. | G9 |
| N13 | 1. | 1. | 1. | G9 |
| N14 | 1. | 1. | 1. | G9 |
| N15 | 1. | 1. | 1. | G9 |
| N16 | 1. | 1. | 1. | G9 |
| N17 | 1. | 1. | 1. | G9 |
| N18 | 1. | 1. | 1. | G9 |
| N19 | 1. | 1. | 1. | G9 |
| N20 | 1. | 1. | 1. | G9 |

It can be seen from the detection results of the above table that if it is a positive result, N+S way is taken; for 100% positive result, a single RBD detection cannot obtain 100% detection rate, resulting in missing detection. It seems to indicate that the N+S detection has high consistency with the result of nucleic acid, having a conformity of 100%. It indicates from another aspect that the N+S combination way or the antibody of mixed antigens is used as a detection line to effectively detect whether the patient is infected with novel coronavirus; while a single RBD antigen is used to detect antibodies in blood, possibly resulting in missing detection, for example, for a P2 sample, the nucleic acid testing is positive; for RBD detection, the result of either IgG or IgM is negative. In case of N+S detection, the result is positive, and strongly positive (color scale is 7.5), indicating that the combined detection of RBD and N+S can overcome the defect of natural missing detection of RBD.

It can be seen from Table 4 that for negative samples N1-N20, the reading of the third test strip was G9, indicating negative, which was consistent with the actual sample; and the result displayed negative, indicating that the concentration of neutralizing antibodies was lower than the effective protection scope.For positive samples P1-P20, most of the readings on the test strips were G2, namely, positive, indicating that the concentration of neutralizing antibodies was within the effective protection scope; the reading of P15 was G1, indicating that the content of neutralizing antibodies was higher; even though there was a higher amount of viral antigens in the body, the patient had stronger autoimmunity to produce enough neutralizing antibodies to resist the virus, indicating that the patient was in a process of gradual recovery; the reading of P12 was G4, indicating that the patient was infected with novel coronavirus and the antibody was positive, but the content of neutralizing antibodies may be negative, which was judged early infection, and there was no enough neutralizing antibodies capable of resisting the virus, and at this time, it was recommended to take measures in time to help patients enhancing disease resistance.Similarly, by making a comparison to the detection results between the first and second test strips of P2, it can be seen that there is a possibility of missing detection in case of the detection by the second test strip; and the third test strip may be used to help avoiding missing detection; meanwhile, the third test strip can further provide the detection results of neutralizing antibodies, so that the patient can judge the progression of disease.

### Example 6: an antibody was used to detect an antigen in a throat swab sample.

A label pad on a first test strip: a immunogold labeled first monoclonal antibody (AB-RBD) against a RBD antigen was treated, and a detection line was immobilized with an AB-RBD antibody of the monoclonal antibody. When the throat swab sample contained the RBD antigen, the first monoclonal antibody bonded the RBD antigen to form an immunogold labeled-AB-RBD-RBD complex substance; when the complex moved to an anti-AB-RBD antibody area of a nitrocellulose membrane, the immunogold labeled-AB-RBD-RBD complex was captured by the anti-AB-RBD antibody to show colored strips. An anti-RBD antigen second monoclonal antibody (a double-antibody sandwich method) may be immobilized in the detection line. Based on the same principle, it may be used to detect an S antigen, or S+N antigen. During the detection of N+S antigen, the immunogold labeled first monoclonal antibody against the S antigen (AB-S) and the immunogold labeled second monoclonal antibody against the N antigen (AB-N) were mixed or respectively labeled, and then sprayed onto a label pad respectively. The detection line was immobilized with the second monoclonal antibody against the S antigen and the second monoclonal antibody against the N antigen. There was a positive result in the detection line as long as the sample contained N or S antigen fragments. To the third test strip: an angiotensin converting enzyme 2 (ACE2) was coated or immobilized on a detection line of a nitrocellulose membrane; and a label pad was coated with a gold-labeled S-RBD antibody (antigen). 10 blood samples infected with novel coronavirus confirmed clinically and 10 negative samples N1-N20 (samples confirmed clinically) were detected to obtain the detection results below.

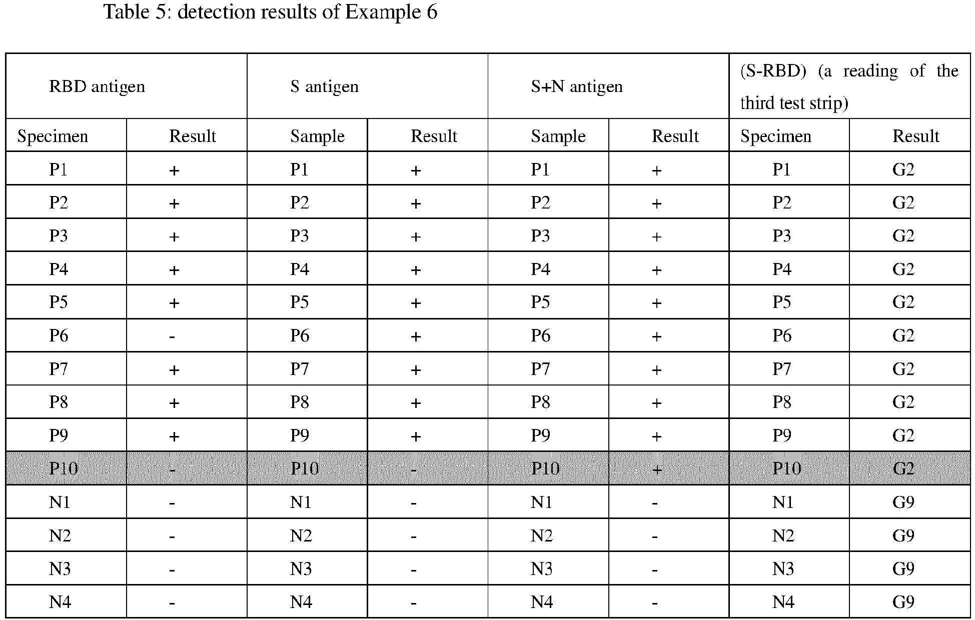

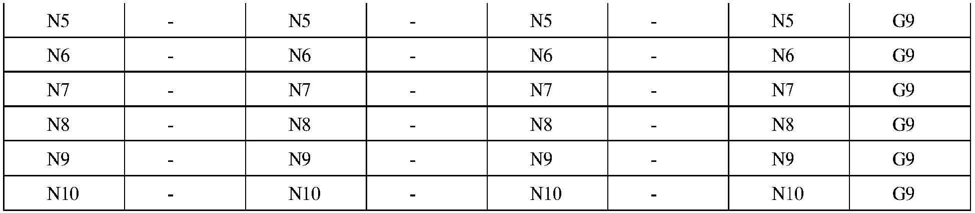

It can be seen from the above detection results that the above 10 positive samples cannot be detected effectively by a RBD or S full-length antigen, resulting in missing detection of samples (the rate of missing detection is nearly 10%); while in case of detection by a N antigen or N+S antigens, there is no missing detection, and for the detection of neutralizing antibodies, there is no missing detection, either. It indicates from another aspect that the detection by a single N antigen may also obtain a 100% detection rate. It seems to indicate that the detection by a single N full-length antigen is more effective than that by a single RBD in the aspect of positive detection rate, meanwhile, a neutralizing antibody is added to help preventing missing detection. However, RBD antibodies or antigens are detected more in actual detection, it may be because people usually believe that RBD is a major infection area to cause infection, and RBD binds an ACE2 area of cells to cause infective cells, but neglect the detection of the N or S full-length sequence; actually, the detection to full-length sequence may detect more sites; and these sites may cause immune responses to stimulate the body to produce antibodies; generally, RBD is regarded as a key site, and meanwhile, separate detection of other protein sites is combined to effectively avoid missing detection to improve the detection rate, thus effectively controlling a contagion risk; moreover, the addition of neutralizing antibodies can help patients to judge the disease progression and reminder whether of getting into rehabilitation, which has very good significance of clinical promotion.

### Example 7: detection effects of blood samples at different stages.

Test strip 1: the test strip was the same as that in Example 2, there were different detection results by monitoring a blood sample at different times. A goat anti-mouse IgG or goat anti-chicken IgY antibody was coated on a quality control line of a nitrocellulose membrane, a mouse anti-human IgG antibody was coated on an IgG detection line; a mouse anti-human IgM antibody was coated on an IgM detection line; and a label pad is coated with a colloidal gold-labeled S protein-RBD (only including the antigen at a RBD site) and a colloidal gold-labeled chicken-IgY antibody (a control line). Detection principle: if there was an antibody (IgG or IgM) of the S protein-RBD in a blood sample, the antigen on a label pad would bind the antibody in a sample to form: S protein-RBD-IgG or IgM-metal particle; then the obtained product was captured by a human IgG antibody immobilized on a detection line, or a human IgM antibody to obtain a particle complex, thus forming a positive or negative result.

**Table 6: detection results of Example 7 (test strip 7)**

| Clinical samples | IgM detection result (positive/sample size) | IgG detection result (positive/sample size) |
|---|---|---|
| 10 early clinical serum specimens (nucleic acid was confirmed positive) | 6-Positive/10 (Positive) | 4-Positive/10 (Positive) |
| 10 medium clinical serum specimens (positive confirmed) | 9-Positive/10 (Positive) | 9-Positive/10 (Positive) |
| 10 later-period clinical serum specimens (positive confirmed) | 7-Positive/10 (Positive) | 9-Positive/10 (Positive) |
| 10 cases-excluded specimens (negative confirmed) | 10 Negative/10 (Negative) | 10 Negative/10 (Negative) |
| 10 whole blood specimens (negative confirmed) | 10 Negative/10 (Negative) | 10 Negative/10 (Negative) |

Antibodies at S-RBD sites were detected only, for 10 positive samples (nucleic acid of a throat swab was confirmed), there was the possibility of missing detection in early stage or medium stage, moreover, the early the stage was, the higher the possibility of missing detection was.

Test strip 2: The test strip was the same as that in Example 2, there were different detection results by monitoring a blood sample at different times.A goat anti-mouse IgG and/or goat anti-chicken IgY antibody was coated on a quality control line of a nitrocellulose membrane, a mouse anti-human IgG antibody was coated on an IgG detection line; a mouse anti-human IgM antibody was coated on an IgM detection line; and a label pad was coated with a colloidal gold-labeled S full-length protein and N full-length protein (antigen) and a colloidal gold-labeled chicken-IgY antibody.

**Table 7: detection results of Example 7 (test strip 2)**

| Clinical samples | IgM detection result (positive/sample size) | IgG detection result (positive/sample size) |
|---|---|---|
| 10 early clinical serum specimens (positive confirmed) | 6-Positive/10 (Positive) | 4-Positive/10 (Positive) |
| 10 medium clinical serum specimens (positive confirmed) | 8-Positive/10 (Positive) | 10-Positive/10 (Positive) |
| 10 later-period clinical serum specimens (positive confirmed) | 7-Positive/10 (Positive) | 10-Positive/10 (Positive) |
| 10 cases-excluded specimens (negative confirmed) | 10 Negative/10 (Negative) | 10 Negative/10 (Negative) |
| 10 whole blood specimens (negative confirmed) | 10 Negative/10 (Negative) | 10 Negative/10 (Negative) |

Detection principle: if there was an antibody (IgG or IgM) of the S or N full-length protein in a blood sample, the antigen on a label pad would bind the antibody in a sample to form: metal particle-S or N full-length protein (antigen)-IgG or IgM (sample); then the obtained product was captured by a human IgG antibody immobilized, or a human IgM antibody to obtain a particle complex, thus forming a positive or negative result (metal particle-S protein or N protein (antigen)-IgG or IgM (sample)-human IgG antibody, or human IgM antibody).

It can be seen from the above results that the detection of S and N full-length proteins is more in line with the actual condition relative to the detection of S-RBD sites only, thus reducing the possibility of missing detection.

Test strip 3: the test strip was the same as that in Example 2, there were different detection results by monitoring a blood sample at different times.A goat anti-mouse IgG was coated on a quality control line on a nitrocellulose membrane, and an S full-length protein was coated on an S detection line; a N full-length protein was coated on a N detection line; and a label pad was coated with a colloidal gold-labeled mouse anti-human IgG and mouse anti-human IgM antibodies.

**Table 8: detection results of Example 7 (test strip 3)**

| Clinical samples | S detection result (positive/sample size) | N detection result (positive/sample size) |
|---|---|---|
| 10 early clinical serum specimens (negative confirmed) | 6-Positive/10 (Positive) | 1-Positive/10 (Positive) |
| 10 medium clinical serum specimens (negative confirmed) | 8-Positive/10 (Positive) | 10-Positive/10 (Positive) |
| 10 later-period clinical serum specimens (negative confirmed) | 7-Positive/10 (Positive) | 10-Positive/10 (Positive) |
| 10 cases-excluded specimens (negative confirmed) | 10 Negative/10 (Negative) | 10 Negative/10 (Negative) |
| 10 whole blood specimens (negative confirmed) | 10 Negative/10 (Negative) | 10 Negative/10 (Negative) |

It can be seen from the above description that the detection of N full-length sequence can serve as a supplementary detection or combined detection of the S-RBD full-length sequence, thus reducing the rate of missing detection.

Detection principle: if there was an antibody (IgG or IgM) of the N full-length protein in a blood sample, the mouse anti-human IgG and mouse anti-human IgM antigens on a label pad would bind the antibody in a sample to form: metal particle-mouse anti-human IgG and mouse anti-human IgM antibody (labeled)-IgG or IgM (sample); then the obtained product was captured by the human N protein immobilized to obtain a particle complex, thus forming a positive or negative result (metal particle-mouse anti-human IgG and mouse anti-human IgM antibodies (labeled)-IgG or IgM (sample)-N protein).

Test strip 4: a goat anti-mouse IgG and/or goat anti-chicken IgY antibody was coated on a quality control line of a nitrocellulose membrane, an S full-length protein was coated on an S detection line; a N protein was coated on a N detection line; and a label pad was coated with a colloidal gold-labeled S full-length protein and N full-length protein and a colloidal gold-labeled chicken-IgY antibody.

**Table 9: detection results of Example 7 (test strip 4)**

| Clinical samples | S detection result (positive/sample size) | N detection result (positive/sample size) | |
|---|---|---|---|
| 10 early clinical serum specimens (positive confirmed) | 6-Positive/10 (Positive) | | 1-Positive/10 (Positive) |
| 10 medium clinical serum specimens (positive confirmed) | 8-Positive/10 (Positive) | | 10-Positive/10 (Positive) |
| 10 later-period clinical serum specimens (positive confirmed) | 7-Positive/10 (Positive) | | 10-Positive/10 (Positive) |
| 10 cases-excluded specimens (negative confirmed) | 10 Negative/10 (Negative) | | 10 Negative/10 (Negative) |
| 10 whole blood specimens (negative confirmed) | 10 Negative/10 (Negative) | | 10 Negative/10 (Negative) |

Detection principle: if there is an antibody of an S protein or a N protein in a sample, the antibody forms: mark-S protein or N protein-antibody (in a sample) on a label pad, and captured by S and N proteins immobilized in a detection area to form: mark-S protein or N protein-antibody (in a sample)-S and N proteins.

Test strip 5: a goat anti-mouse IgG and/or goat anti-chicken IgY antibody was coated on a quality control line of a nitrocellulose membrane; the detection line was coated with an S full-length protein (antigen) and a N full-length protein (antigen); and a label pad was coated with a colloidal gold-labeled S full-length protein, S full-length protein and a colloidal gold-labeled chicken IgY antibody.

**Table 10: detection results of Example 7 (test strip 5)**

| Clinical samples | Detection result (positive/sample size) |
|---|---|
| 10 early clinical serum specimens (positive confirmed) | 6-Positive/10 (Positive) |
| 10 medium clinical serum specimens (positive confirmed) | 10-Positive/10 (Positive) |
| 10 later-period clinical serum specimens (positive confirmed) | 10-Positive/10 (Positive) |
| 10 cases-excluded specimens (negative confirmed) | 10 Negative/10 (Negative) |
| 10 whole blood specimens (negative confirmed) | 10 Negative/10 (Negative) |

S and N are detected at the same time, which may reduce the rate of missing detection under the detection of a single S-RBD, so that the detection result is more close to the real situation.

Test strip 6: a goat anti-mouse IgG and/or goat anti-chicken IgY antibody was coated on a quality control line of a nitrocellulose membrane; the detection line (T) was coated with an S full-length protein; and a label pad was coated with a colloidal gold-labeled S full-length protein, and a colloidal gold-labeled chicken IgY antibody.

**Table 11: detection results of Example 7 (test strip 6)**

| Clinical samples | Detection result (positive/sample size) |
|---|---|
| 10 early clinical serum specimens (positive confirmed) | 6-Positive/10 (Positive) |
| 10 medium clinical serum specimens (positive confirmed) | 8-Positive/10 (Positive) |
| 10 later-period clinical serum specimens (positive confirmed) | 7-Positive/10 (Positive) |
| 10 cases-excluded specimens (negative confirmed) | 10 Negative/10 (Negative) |
| 10 whole blood specimens (negative confirmed) | 10 Negative/10 (Negative) |

Test strip 7: a goat anti-mouse IgG and/or goat anti-chicken IgY antibody was coated on a quality control line of a nitrocellulose membrane; the detection line (T) was coated with a N full-length protein (antigen); and a label pad was coated with a colloidal gold-labeled N full-length protein, and a colloidal gold-labeled chicken IgY antibody.

**Table 12: detection results of Example 7 (test strip 7)**

| Clinical samples | Test results |
|---|---|
| 10 early clinical serum specimens (positive confirmed) | 6-Positive/10 (Positive) |
| 10 medium clinical serum specimens (positive confirmed) | 8-Positive/10 (Positive) |
| 10 later-period clinical serum specimens (positive confirmed) | 7-Positive/10 (Positive) |
| 10 cases-excluded specimens (negative confirmed) | 10 Negative/10 (Negative) |
| 10 whole blood specimens (negative confirmed) | 10 Negative/10 (Negative) |

Test strip 8: an angiotensin converting enzyme 2 (ACE2) was coated or immobilized on a detection line of a nitrocellulose membrane; and a label pad was coated with a gold-labeled S-RBD antibody (antigen).

**Table 12: detection results of Example 7 (test strip 8)**

| Clinical samples | Test results |
|---|---|
| 10 early clinical serum specimens (positive confirmed) | 1-Positive/10 (Positive) |
| 10 medium clinical serum specimens (positive confirmed) | 10-Positive/10 (Positive) |
| 10 later-period clinical serum specimens (positive confirmed) | 10-Positive/10 (Positive) |
| 10 cases-excluded specimens (negative confirmed) | 10 Negative/10 (Negative) |
| 10 whole blood specimens (negative confirmed) | 10 Negative/10 (Negative) |

The above test strips may be used separately for detection, or test strip 1 may be combined with test strips 2-7, and test strip 8 to obtain three combinations for joint detection, as shown in FIG. 1 to FIG. 4. The above experiments may at least indicate that the detection for the S or N full-length protein can serve as an effective supplementary to detect S-RBD only, which can effectively reduce the possibility of missing detection, conforming to the real situation; the detection for neutralizing antibodies may also serve as an effective supplementary to detect S-RBD, which can effectively reduce the possibility of missing detection, and can help patients self-detecting and judging the recovery and vaccine immunity condition, which provides more visual and reliable self-checking means for the patients to perform self-screening, control the progression and recovery of disease, or control the effect of the vaccine, and judge whether of requiring supplementary vaccination. Moreover, the test device has the following advantages of rapid detection speed and simple operation, is free from professional instrument and personnel, and easy to clinical popularization and application.

All patents and publications mentioned in the description of the present invention are technical disclosures in the art, and can be used in the present invention. All patents and publications quoted therein are also listed in the references, which is the same as the single quotation of each publication specifically. The present invention herein may be achieved in case of lacking any one or more elements, one or more limiting which are not stated specifically herein. For example, the terms "comprising", "consisting of ...substantively" and "consisting of ..." in each example herein may be replaced by the rest 2 terms. The so-called "one" herein merely denotes its literal meaning "a/an", but does not exclude including only one, and may further denote including 2 and above. The terms and means of expression herein are not intended to be limiting in any way; and there is no intention to indicate that these terms and explanation described in the description exclude any equivalent feature, but it should be understood that any suitable change or modification can be made within the spirit and scope of the present invention and claims. It should be understood that embodiments described in the present invention are preferred examples and features; and any person skilled in the art can make some changes and alterations according to the spirit of the present invention; moreover, these changes and alterations are regarded to be within the scope of the present invention and the scope of the independent claims and dependent claims.

## Claims

1. A lateral flow test device for detecting a coronavirus antibody by immunoassay, **characterized by** comprising a first test strip, a second test strip and a third test strip; wherein the first test strip comprises an S full-length protein antigen and/or a N full-length protein antigen; the second test strip comprises an S-RBD-site protein antigen; or the first test strip comprises an antibody for binding an S full-length protein antigen and/or a N full-length protein; the second test strip comprises an antibody for binding an S-RBD-site protein antigen; and the third test strip comprises an S-RBD-site protein antigen and angiotensin converting enzyme 2(ACE2).

2. The test device according to claim 1, **characterized in that** the S full-length protein has an amino acid sequence as shown in SEQ ID NO.1; the N full-length protein has an amino acid sequence as shown in SEQ ID NO.2, and the S-RBD-site protein has an amino acid sequence as shown in SEQ ID NO.3.

3. The test device according to claim 2, **characterized in that** the first test strip, the second test strip, and the third test strip respectively comprise a sample area, a label area and a detection area, the sample area, the label area and the detection area being arranged successively according to a direction of liquid flow; wherein a substance coated in the label area may flow with liquid and the substance needs to be coupled with a marker; the detection area is provided with a detection line; and a substance coated in the detection area needs to be immobilized on the detection line; and the antigen may be coated in the label area.

4. The test device according to claim 3, **characterized in that** an anti-human IgG antibody and/or anti-human IgM antibody are immobilized on detection areas of the first and the second test strips; an angiotensin converting enzyme 2 is immobilized on a detection area of the third test strip; and when the antigen is coupled to a marker with colored particles and treated on the label area; or when the antigen is immobilized on the detection area, the anti-human IgG antibody and/or anti-human IgM antibody and/or the angiotensin converting enzyme 2 are coupled to the marker with colored particles and coated on the label area.

5. The test device according to claim 4, **characterized in that** the detection areas of the first and the second test strips may be provided with a first or second detection line; and the first and the second detection lines are respectively used to detect any one or two of IgG and IgM antibodies in a sample.

6. The test device according to claim 4, **characterized in that** the S-RBD-site protein antigen on the second test strip is coated in a label area; the anti-human IgG and anti-human IgM antibodies are respectively immobilized on the first and the second detection lines of the detection area.

7. The test device according to claim 5, **characterized in that** the N or/and S protein antigens on the first test strip are coated in a label area; the anti-human IgG and anti-human IgM antibodies are respectively immobilized on the first and the second detection lines of the detection area; or the anti-human IgG and anti-human IgM antibodies are immobilized on the first or the second detection line of the detection area simultaneously.

8. The test device according to claim 1, **characterized in that** the antibody comprises a first antibody or a second antibody; when a first anti-S full-length protein antibody and/or a second anti-N full-length protein antibody are treated on a label area of the first test strip, a second antibody against an S full-length protein and/or a N full-length protein antibody is immobilized on a detection area.

9. The test device according to claim 1, **characterized in that** the antibody comprises a first antibody or a second antibody; wherein the first antibody is used to bind an antigen in a sample and is treated on a label area; and the second antibody is an antibody of a first antibody and is immobilized on a detection area.

10. The test device according to claim 3, **characterized in that** the detection area is further provided with a quality control line.

11. The test device according to claim 3, **characterized in that** the first test strip, the second test strip, or the third test strip further comprises a water absorbing area, used for adding a buffer solution.

12. The test device according to claim 1, **characterized in that** the coronavirus is a novel coronavirus.
